# EUROPEAN PATENT APPLICATION

(11) **EP 2 684 960 A1**
(43) Date of publication of application: **15.01.2014**
(21) Application number: 12005108.1
(22) Date of filing: 10.07.2012
(51) Int. Cl.: C12N 15/82, C12Q 1/66

(54) **Expression vector for a secretion and detection system**

(71) Applicant: Universität Bielefeld, 33615 Bielefeld (DE)
(72) Inventor: Kruse, Olaf, Prof. Dr., 33605 Bielefeld (DE); Lauersen, Kyle Jonathan, 33615 Bielefeld (DE); Mussgnug, Jan Hermann, Dr., 33613 Bielefeld (DE)
(74) Representative: Lahrtz, Fritz

(57) **Abstract**

The present invention relates to an expression vector comprising an Open Reading Frame (ORF) comprising sequences encoding for a *Chlamydomonas* carbonic anhydrase I (CAH1) secretion signal, a target polypeptide and a luciferase. Furthermore, the present invention refers to a host cell comprising said expression vector and to the use of said cells for producing a polypeptide.

## Description

The present invention relates to an expression vector comprising an Open Reading Frame (ORF) comprising sequences encoding for a *Chlamydomonas* carbonic anhydrase 1 (CAH1) secretion signal, a target polypeptide and a luciferase. Furthermore, the present invention refers to a host cell comprising said expression vector and to the use of said cells for producing a polypeptide.

The production of recombinant proteins has gained increasing importance in scientific and applied research, medical and industrial applications over the past several decades. Today, recombinant proteins are found in many products and are used in numerous applications. Exemplarily, numerous recombinant proteins serve as therapeutic and diagnostic agents, as food and feed ingredients, as ingredients in laundry agents and in dishwashers, as anti-freezing agents and even as additives in concrete, paints, fibers, fabrics and composite materials.

The wide use of recombinant polypeptides is limited by still insufficient means of production, namely, the expression of the polypeptides in host cells. Depending on the chemical structure of the recombinant polypeptide to be expressed, the most often used bacterial host cells generally have the considerable drawback that polypeptides of eukaryotic origin are not folded correctly and that posttranslational modifications may differ from the posttranslational modifications found for a polypeptide strand expressed in a eukaryotic host cell. It is well known that these differences are of some importance for the activity and immunogenicity of therapeutic, diagnostic and ingestible polypeptides and vaccines.

Furthermore, the vast majority of usable host cells, in particular eukaryotic host cells, require complex media comprising sugars and amino acids for their vitality and to produce the recombinant polypeptide. Widely used animal and human host cells generally require high-molecular weight growth factors such as serum-derived growth factors. Sugars, amino acids and growth factors often cause impurities that are even known to be able to cause severe immunogenic issues.

In the view of the above, an expression system is needed that is usable in a eukaryotic cell extensively autologous with respect to sugars, amino acids and that is extensively independent of growth factors.

It has been shown that plant cells, in particular alga cells, can be used for the production of recombinant proteins and overcome most of the above problems. In particular, microalgae are considered for this purpose. Microalga cultures are comparably easy to handle and, in principle, scalable to large scale productions. Further, microalgae merely require a slightly salty aqueous environment, CO₂ and light, can be supplemented by simple carbon sources, or a combination of these strategies to grow rapidly and produce the recombinant protein of interest. Therefore, the cultivation of microalgae is relatively inexpensive. These cultures can be extensively free of sugars and amino acids. The use of solar energy to produce biomass and recombinant protein product is indeed an attractive aspect of these cells in light of the goal of sustainability. In principle, microalgae are valuable hosts for the production of recombinant polypeptides in addition to their current industrial use for the production of a variety of other high value products ranging from dyes and pigments to antioxidants and omega 3 fatty acids.

However, the yields of polypeptide production in microalgae to date have been comparably low. The efficient production of recombinant, nuclear encoded proteins in microalgae is hampered because of the tightly regulated gene expression, preference for certain nucleic acid codon usage, and bias to its own promoters. Some technical limitations surrounding nuclear recombinant protein expression in microalgae of complex folded eukaryotic proteins have been recently overcome (Sprecht, et al. 2010).

Nevertheless, efficient production is still severely hampered as the initial detection of high-quantity polypeptide-producing transformants is difficult, time-consuming and recombinant polypeptide purification is laborious and expensive. In fact, one of the most severe drawbacks of expressing a polypeptide recombinantly still is that, after expression, the polypeptide is typically located in the cytoplasm of the host cells and must be isolated from said cytoplasm by separating the expressed recombinant polypeptide from hundreds or even thousands of other polypeptides that are present in the cytoplasm concomitantly. Typically, this isolation is laborious, time-consuming and expensive. For many purification techniques further impurities are included in the system, such as toxic solvents and salts when using chromatographic and/or precipitation methods. This challenge is met with several approaches, besides others, by the use of recombinant secretion mechanisms (Eichler-Strahlberg *et al.,* 2009).

However, these methods do not inherently facilitate the fast and easy identification of the high polypeptide producing transformant host cells concurrently with the cultivation of the cells. In particular, these methods do not allow the observation of polypeptide production concomitantly to the expression of the polypeptide in the host cells without harming the cells. These pieces of information, however, would be of large benefit for the efficient selection of suitable host cells and/or for the identification of the time point for isolating the polypeptide from the culture broth.

There is always a need for efficient expression vectors.

In a first aspect, the present invention relates to an expression vector comprising an Open Reading Frame (ORF) comprising sequences encoding for:
a) a *Chlamydomonas* carbonic anhydrase 1 (CAH1) secretion signal;
b) a target polypeptide; and
c) a luciferase.

As shown in the examples, the expression vector of the present invention surprisingly allows highly efficient production of target polypeptides, secretion of said peptides, and enables monitoring of the polypeptide production without disturbing the expression system.

As used in the context of the present invention, the term "expression vector" may refer to any molecular structure conveying genetic information. The genetic information may be encoded as deoxyribonucleic acid (DNA) (double stranded DNA (dsDNA), single stranded DNA (ssDNA)), ribonucleic acid (RNA), (single-stranded RNA (ssRNA), double-stranded RNA (dsRNA)), or as a DNA analog such as, e.g., peptide nucleic acid (PNA), morpholino, glycol nucleic acid (GNA), threose nucleic acid (TNA) or methylated DNA. Preferably, the genetic information is encoded as DNA. The vector may be any vector known in the art such as, e.g., a linear vector, a circular vector, a viral vector or a bacterial vector.

In a preferred embodiment, the expression vector is a nucleic acid vector, in particular a linear or circular DNA vector.

The encoding sequences of the expression vector may be expressible in an extracellular expression system and/or in a host cell. Preferably, the vector is expressible in a host cell, most preferably in a host cell as defined below. The term "expressed" may be understood in the broadest sense as the conversion of genetic information into a polypeptide strand.

Therefore, when the vector is a DNA vector, the term "expression" as used herein includes the steps of transcribing the DNA into mRNA and translating said mRNA into a polypeptide strand. These processes are well-known by those skilled in the art. When the vector is an RNA vector, the term "expression" as used herein includes at least the step of translating said mRNA into a polypeptide strand. Optionally, there may or may not be step(s) of splicing the mRNA and/or postranslationally modifying said polypeptide strand.

As used herein, the term "Open Reading Frame" and the abbreviation "ORF" may be understood in the broadest sense a part of a gene that encodes a polypeptide strand. The ORF is located between a start codon and a stop codon. The person skilled in the art will know that the polypeptide-encoding sequence of an ORF is not interrupted by any stop or termination pause site and that each ORF encodes for one continuous polypeptide strand each. The transcription termination pause site is located after the ORF, beyond the stop codon. Herein, the ORF comprises an expression cassette for fusion of the CAH1 secretion signal (CA) with at least one target polypeptide and a luciferase.

In the view of the present invention, expression of an ORF of the expression vector according to the present invention results in a single polypeptide strand comprising:
a) a *Chlamydomonas* CAH1 secretion signal;
b) a target polypeptide; and
c) a luciferase.

Therefore, upon expression, a fusion polypeptide comprising domains serving as a CAH1 secretion signal, target polypeptide and luciferase is obtained. The CAH1 secretion signal may typically be removed during the secretion process. Therefore, optionally, depending on the individual sequence, a polypeptide comprising a target polypeptide and a luciferase without the CAH1 secretion signal may finally be obtained.

As used in the context of the present invention, the terms "polypeptide", "polypeptide strand", "peptide" and "protein" may be understood interchangeably in the broadest sense as a linear or branched polymer mainly composed of amino acids. The polypeptide of the present invention is a recombinant polypeptide. Typically, the polypeptide will be at least four amino acids long. The polypeptide may also be more than ten, more than 20, more than 30, more than 40, more than 50, more than 75, more than 100, more than 150, more than 200, more than 300, more than 400 or even more than 500 amino acids in length. The person skilled in the art will immediately notice that, during and/or after expression, a polypeptide may optionally be subjected to one or more posttranslational modification(s). Posttranslational modifications are well-known in the art and may comprise but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, nitration, nitrosylation, disulfide bond formation, pyroglutamate formation, amino acid addition, cofactor addition (e.g., biotinylation, heme addition) and complexation of metal ions, non-metal ions, peptides or small molecules and addition of iron-sulfide clusters. Further, co-factors, such as, e.g., ATP, ADP, NAD+, NADH+H⁺, NADP⁺, NADPH+H⁺, metal ions, cations, anions, lipids may be bound to the polypeptide, irrespective on their biological impact. Moreover, the protein may be elongated by one or more amino acids.

It will be understood that an ORF of the expression vector according to the present invention may also encode for two, three, four or more target polypeptides that can be located in a polypeptide strand in any sequential order. Exemplarily, in addition to a sequence encoding for a target polypeptide of interest, the same ORF may also encode for a one or more protein tag(s) such as, e.g., polyhistidine (polyHIS) tag(s), dihydrofolate, polyhistidine tag(s), streptavidin, or others that may have a function in polypeptide purification.

The *Chlamydomonas* carbonic anhydrase 1 (CAH1) secretion signal according to the present invention may be any CAH1 secretion signal as found throughout the population of any *Chlamydomonas* species and serves as transporter for inorganic carbon uptake. Exemplarily, a *Chlamydomonas* species in the sense of the present invention may be *Chlamydomonas reinhardtii* (*C. reinhardtii*), *Chlamydomonas caudata* Wille (*C. caudata* Wille), *Chlamydomonas moewusii* (*C. moewusii*), *Chlamydomonas nivalis* (*C. nivalis*) or *Chlamydomonas dysosmos* (*C. dysosmos*), or others. As used herein, the terms "carbonic anhydrase" and "carboanhydrase" may be understood interchangeably.

The CAH1 secretion signal leads to the secretion of the protein from a host cell expressing the polypeptide into the medium that may allow easier and improved isolation and purification, because harvesting the cells and disrupting said cells may be dispensable. As used herein, "secretion" means that the polypeptide is transported out of the cell and is then localized extracellularly, i.e., in the medium outside the cells (extracellular and/or periplasmic space). Polypeptide secretion may occur by gene expression followed by protein translation and subsequent targeting to the endoplasmic reticulum (ER). From the ER, proteins are transported through known mechanisms to the cell membrane and localized outside of the cell.

Furthermore, masking the recombinant polypeptide with the CAH1 secretion signal may have the advantage of providing additional stability for the product.

The term "throughout the population" expressly includes minor genetic modifications throughout the population, in particular single nucleotide polymorphisms (SNPs) as well as potential codon use biases. Furthermore, the CAH1 secretion signal may be a truncated from of CAH1 secretion signal having at least four, at least five, at lest six, at least seven, at least eight, at least nine, at least ten, at least eleven, at least twelve, at least 13, at least 14, at least 15, at least 16, at least 17, at least 18, at least 19, at least 20 or 21 amino acids. Furthermore, the CAH1 secretion signal may be an elongated CAH1 secretion signal wherein having one, two, three, four, five, six, seven, eight, nine, ten or more amino acids added by molecular biological means.

In a preferred embodiment, the CAH1 secretion signal has a sequence homology of at least 60 % to SEQ ID NO: 1.

SEQ ID NO: 1 represents the amino acid sequence:
MARTGALLLVALALAGCAQAC

This amino acid sequence has been derived from the amino acid sequence of amino acid positions 1-21 of the wild type CAH1 from *Chlamydomonas reinhardtii.* The corresponding full-length polypeptide is encoded by SEQ ID NO: 18 (Fukuzawa *et al.,* 1990).

Even more preferably, the CAH1 secretion signal has a sequence homology of at least 70 %, even more preferably at least 80 %, even more preferably at least 90 % to SEQ ID NO: 1 or may be, most preferably identical to SEQ ID NO: 1.

Alternatively, the CAH1 secretion signal is encoded by a nucleic acid having a sequence homology of at least 40 %, a sequence homology of at least 50 %, a sequence homology of at least 60 %, more preferably at least 70 %, even more preferably at least 80 %, even more preferably at least 90 %, even more preferably at least 95 % to SEQ ID NO: 2 or may be, most preferably, identical to SEQ ID NO: 2.

SEQ ID NO: 2 represents the nucleic acid sequence:

ATGGCGCGTACTGGCGCTCTACTCCTGGTCGCGCTGGCGCTTGCGGGCT
GCGCGCAGGCTTGC

The target polypeptide may be any polypeptide that may be of interest to be expressed.

In a preferred embodiment, the target polypeptide is an anti-freeze peptide, a food ingredient, a pharmaceutical ingredient, a bioremediation or industrial agent, a therapeutic agent, a diagnostic agent or a vaccine.

Highly preferably, the target polypeptide may be an anti-freeze peptide. As used herein, the terms "anti-freeze peptide", "antifreeze peptide", "anti freeze peptide", "anti-freeze protein", "antifreeze protein", "anti freeze protein", "AFP", "ice structuring protein", "ice structuring peptide", "ISP", "ice binding protein", "IBP", "ice recrystallization inhibition protein" and "IRI" may be understood interchangeably as a target polypeptide that binds to small ice crystals and thereby inhibits the formation, growth and recrystallization of ice. Alternatively, a similar class of ice binding peptides with the reverse function may also be targeted; the so-called "ice nucleation proteins" or "INP" (e.g., from *Pseudomonas syringae*). Therefore, an anti-freeze peptide may lead to a rather amorphous crystallization that is typically less harmful to organisms and machines. Exemplarily, anti-freezing peptides may be used in food (e.g., in (deep-)frozen food to maintain the food texture (e.g., in and/or on frozen vegetables or frozen processed food such as, e.g., (fancy) cake, ice cream, frozen fish, frozen seafood, frozen meat, frozen edible snails) or food of which an intermediate product is frozen (e.g. for transportation and/or storage), in freeze-dried food (e.g., powders of soup, spices or beverages)), for freezing or freeze-drying biological material (e.g., cell or tissue cultures, polypeptides (e.g., enzymes, antibodies, antibiotics), vaccines, fats), to protect machines facing cold environments (e.g., vehicles (e.g. cars, agricultural vehicles, ships, trains), plains, chemical production plants, power plants, drilling devices), an additive to prevent clathrate hydrate formation in liquid hydrocarbons (e.g., gasoline, diesel, crude oil, or other liquid hydrocarbons) and/or as de-icer.

Anti-freeze peptides are known to be produced in nature by certain vertebrates, plants, fungi and bacteria and may permit their survival in subzero environments. Anti-freeze peptides are also known to interact with mammalian cell membranes to protect them from cold damage and have impact in cold acclimatization. An anti-freeze peptide may serve as freeze avoidant, i.e., by lowering the freezing point, and/or as a freeze tolerant, i.e., as a cryoprotectant preventing the damages of freezing, but not freezing altogether. An anti-freeze peptide may originate from any organism encoding anti-freeze peptides. Exemplarily, an anti-freeze peptide may originate from a plant (e.g., *Lolium perenne*) Antarctic notothenioids, winter flounder, longhorn sculpin and shorthorn sculpin, righteye flounders, sea raven, smelt and herring, Antarctic eelpout, *Tenebrio, Dendroides, Choristoneura fumiferana, Fragilariopsis cylindrus, Fragilariopsis curta Colwellia spp., Navicula glaciei, Chaetoceros neogracile, Stephos longipes* and *Leucosporidium antarcticum* and Antarctic inland ice bacteria (e.g., *Flavobacteriaceae*) and cold-tolerant fungi (e.g, *Typhula ishikariensis, Lentinula edodes, Flammulina populicola*) and insects (e.g. *Choristoneura* or Spruce Budworm). As used throughout the present invention, the expression that an anti-freeze peptide originates from a certain species should not be understood in a way that the respective molecules themselves (i.e., the expression vector, a fraction thereof or the target polypeptide or fraction thereof) necessarily has to originate from said species, but rather in a way that it bears a sequence originating from said species.

Exemplarily, the anti-freeze peptide is the ice binding domain of the *Lolium perenne* ice binding protein (*Lp*IBP) (Lauersen et al., 2011). As used herein, the terms "*Lolium perenne* ice binding protein", "*Lp*IBP", "*Lolium perenne* anti-freeze protein" and "*Lp*AFP" may be understood interchangeably. This protein can be isolated and purified by any means known in the art and exemplified herein.

As used herein, the term "food" includes every ingestible composition, in particular any human and animal foodstuff (e.g., processed nutrition, animal feed) and any beverage (e.g., soft drink, alcoholic drink). When the target polypeptide is used as food ingredient or pharmaceutical ingredient, it may or may not have influence on the taste perception of the food or pharmaceutical product (e.g., as sweetening agent, as sweetness enhancer, as umami-enhancer), may or may not have influence on the smell perception of the food or pharmaceutical product, may or may not have anti-freezing activity as described above, may or may not have preserving activity (e.g., as anti-microbial peptide, as antioxidative target polypeptide, as metal-binding target polypeptide (e.g., as ferrin or ferricin (e.g. lactoferrin or lactoferricin)), may or may not have influence on the food or pharmaceutics' texture, may or may not have influence as coloring agent.

When the target polypeptide is used as food or pharmaceutical ingredient, the composition may further comprise one or more further additives next to the food itself. Such additive may exemplarily a non-toxic solvent such as, e.g., water, dimethyl sulfoxide (DMSO), ethanol, vegetable oil, paraffin oil or combinations thereof. Furthermore, a carrier may contain one or more detergents, one or more foaming agents (e.g., sodium lauryl sulfate (SLS)/ sodium doceyl sulfate (SDS)), one or more colouring agents (e.g., TiO₂, food colouring), one or more vitamins, one or more salts (e.g., sodium, potassium, calcium, zinc salts), one or more humectants (e.g., sorbitol, glycerol, mannitol, propylene glycol, polydextrose), one or more enzymes, one or more preserving agents (e.g., benzoic acid, methylparabene), one or more texturing agents (e.g., carboxymethyl cellulose (CMC), polyethylene glycol (PEG), sorbitol), one or more emulsifiers, one or more bulking agents, one or more glazing agents, one or more separating agents, one or more antioxidants, one or more herbal and plant extracts, one or more stabilizing agents, one or more polymers (e.g., hydroxypropyl methacrylamide (HPMA), polyethylene imine (PEI), carboxymethyl cellulose (CMC), polyethylene glycol (PEG)), one or more uptake mediators (e.g., polyethylene imine (PEI), dimethyl sulfoxide (DMSO), a cell-penetrating peptide (CPP), a protein transduction domain (PTD), an antimicrobial peptide, etc.) one or more antibodies, one or more sweeteners (e.g., sucrose, acesulfame K, saccharin Na, stevia), one or more counterstain dyes (e.g., fluorescein, fluorescein derivatives, Cy dyes, an Alexa Fluor dyes, S dyes, rhodamine, quantum dots, etc.), one or more homeopathic ingredients one or more gustatory substances and/or one or more fragrances.

When the target polypeptide is used as therapeutic agent, this means that it has a medicinal activity. Therefore, then the target polypeptide is used in a method for treating or preventing a disease in a patient suffering from said disease.

When the target polypeptide is used as diagnostic agent, this means that it can be used to detect or characterize a disease or condition. Therefore, then the target polypeptide is used in a method for diagnosing a disease or condition.

When the target polypeptide is used as therapeutic or diagnostic agent, the peptide may further comprise a pharmaceutically acceptable carrier. A pharmaceutically acceptable carrier according the present invention may be any additive that is pharmaceutically acceptable, therefore, any additive that is non-toxic to the patient. Exemplarily, a pharmaceutically acceptable carrier may comprise a solvent such as, e.g., water, dimethyl sulfoxide (DMSO), ethanol, vegetable oil, paraffin oil or combinations thereof. Furthermore, a carrier may contain one or more detergents, one or more foaming agents (e.g., sodium lauryl sulfate (SLS)/ sodium doceyl sulfate (SDS)), one or more colouring agents (e.g., TiO₂, food colouring), one or more vitamins, one or more salts (e.g., sodium, potassium, calcium, zinc salts), one or more humectants (e.g., sorbitol, glycerol, mannitol, propylene glycol, polydextrose), one or more enzymes, one or more preserving agents (e.g., benzoic acid, methylparabene), one or more texturing agents (e.g., carboxymethyl cellulose (CMC), polyethylene glycol (PEG), sorbitol), one or more emulsifiers , one or more bulking agents, one or more glacing agents, one or more separating agents, one or more antioxidants, one or more herbal and plant extracts, one or more stabilizing agents, one or more polymers (e.g., hydroxypropyl methacrylamide (HPMA), polyethylene imine (PEI), carboxymethyl cellulose (CMC), polyethylene glycol (PEG)), one or more uptake mediators (e.g., polyethylene imine (PEI), dimethyl sulfoxide (DMSO), a cell-penetrating peptide (CPP), a protein transduction domain (PTD), an antimicrobial peptide, etc.) one or more antibodies, one or more sweeteners (e.g., sucrose, acesulfame K, saccharin Na, stevia), one or more counterstain dyes (e.g., fluorescein, fluorescein derivatives, Cy dyes, an Alexa Fluor dyes, S dyes, rhodamine, quantum dots, etc.), one or more homeopathic ingredients one or more gustatory substances and/or one or more fragrances.

The combination of the target polypeptide as therapeutic agent with a pharmaceutical acceptable carrier may also be understood in the broadest sense as drug or medicine.

When the target polypeptide is used as a vaccine, it may comprise the epitope to be targeted, either as a sequential epitope, as a structural epitope or as a hidden epitope, or may serve as a haptene forming the epitope to be targeted in combination with a small molecule or another high-molecular weight compound.

The target polypeptide may also be a diagnostic tool, such as, e.g., a fluorescence protein (e.g., a green fluorescence protein (GFP)) a fusion protein bound to a fluorescent-labeled protein, an antibody for immunodetection, a radioactively labeled binding compound, a spin-labeled compound. Exemplarily, a diagnostic tool may be used on laboratory scale or for molecular imaging *in vitro* and in a patient *in vivo.*

The target polypeptide may also be an enzyme. An enzyme may be used for the production of chemical compounds, breakdown of compounds, or as a diagnostic tool. The target polypeptide may also be a target polypeptide having antibiotic activity such as, e.g., antibacterial (bactericidal), antiviral, antifungal (fungicidal), insecticidal, herbicidal and/or nematicidal activity. Therefore, the target polypeptide may exemplarily be a small bacterial defense protein. The target polypeptide may also be a recombinant antibody or antibody fragment (e.g., single chain antibody, tandab, diabody, Fc fragment, Fab fragment). The target polypeptide may be a binding ligand (activating, deactivating or neutral; competitively, non-competitively or uncompetitively binding; covalently or non-covalently binding). The target polypeptide may also be recombinant hormone. The target polypeptide may also be a cell-penetrating peptide (CPP) or protein transduction domain (PTD) or may be an enzyme label (e.g., penicillinase, horseradish peroxidase, alkaline phosphatase). The target polypeptide may also be a biosimilar of one of the aforementioned. It may reflect a naturally occurring target polypeptide, a mutant thereof, a truncated form thereof, a fusion target polypeptide or an artificial, synthetic target polypeptide.

Optionally, the target polypeptide may also be immobilized on a solid support such as, e.g., a micro- or nanobead (e.g., a functionalized silica bead, a polysaccharide-based bead), a polymersome, a micelle, a liposome and/or a microarray surface (e.g., a glass surface or a hydrogel surface).

Optionally, the target polypeptide may also serve as ingredient in a laundry agent and in a dishwasher and/or as additive in concrete, a paint, a fiber, a fabric and/or a composite material. The person skilled in the art will further notice that any other target polypeptide may also be the target polypeptide.

As used herein, the term "luciferase" may be understood in the broadest way as an oxidative enzyme that can be used in bioluminescence. Preferably, the luciferase is a modified luciferase, i.e., a luciferase not identical with the corresponding luciferase found in nature. A modified protease may have less than 10%, at least 10%, at least 20%, at least 30%, at least 40%, at least 50%, at least 60%, at least 70%, at least 80%, at least 90% or at least 95% sequence homology to the corresponding luciferase found in nature. Additionally or alternatively, modified luciferase may also be **characterized in that** it comprises one, two, three, four, five, six, seven, eight, nine, ten or more point mutation(s) in comparison to the corresponding natural polypeptide. Additionally or alternatively, modified luciferase may also be **characterized in that** it is truncated by one, two, three, four, five, six, seven, eight, nine, ten or more amino acids at the N-terminus or C-terminus in comparison to the corresponding natural polypeptide. Additionally or alternatively, modified luciferase may also be **characterized in that** it is elongated by one, two, three, four, five, six, seven, eight, nine, ten or more amino acids at the N-terminus or C-terminus in comparison to the corresponding natural polypeptide.

Exemplarily, the luciferase may be derived from crustacean luciferase or may be a modified luciferase derived from crustacean luciferase. Herein, the crustacean luciferase may exemplarily be *Gaussia* luciferase (e.g., from one of the marine copepods *Gaussia princeps, Gaussia asymmetrica, Gaussia gadusae, Gaussia intermedia, Gaussia melanotica* or *Gaussia sewelli*).

Alternatively, the luciferase may be derived from or may be a modified luciferase derived from a luciferase selected from the group consisting of insect luciferase (e.g., from firefly (e.g., *Photinus pyralis*) or click beetles), luciferase from *Renilla reniformis,* mushroom luciferase (e.g. from *Omphalotus olearius*) or dinoflagellate luciferase.

As used throughout the present invention, the definition that a luciferase originates from a certain species or is from a certain species should not be understood in a way that the respective molecules themselves (i.e., the expression vector, a fraction thereof or the polypeptide or fraction thereof) necessarily has to originate from said species, but rather in a way that it bears a sequence encoding for a luciferase originating from said species.

The luciferase marker may enable fast and easy identification of high polypeptide producing host cell lines after transformation with the expression vector of the present invention via bioluminescence assays. This identification may be performed on solid medium and may make technically limiting liquid-culture polypeptide abundance screenings dispensable.

In a preferred embodiment, the luciferase has a sequence homology of at least 60 % to SEQ ID NO: 3.

Expression of gLuc from *Gaussia princeps* in *C. reinhardtii* was described by two groups in 2008 (Ruecker *et al.,* 2008; Shao and Bock, 2008). This protein interacts with the chemical coelenterazine in an adenosine triphosphate (ATP)-independent manner which results in the generation of light with an emission maximum at approximately 485 nm and generation of CO₂. gLuc has been identified as a very robust reporter that is superior of the *Renilla reniformis* reporter.

As used herein, preferably, *Gaussia* luciferase is a modified form of the wild type gLuc, more preferably a truncated form of the wild type gLuc, even more preferably wild type gLuc truncated at the N-terminus. Herein, a truncation at the N-terminus is preferably a truncation wherein the native secretion signal is extensively or completely removed. Therefore, preferably at least the N-terminal amino acids 1-5 are removed, more preferably at least the N-terminal amino acids 1-10 are removed, even more preferably at least the N-terminal amino acids 1-11 are removed, even more preferably at least the N-terminal amino acids 1-12 are removed, even more preferably at least the N-terminal amino acids 1-13 are removed, even more preferably at least the N-terminal amino acids 1-14 are removed, even more preferably at least the N-terminal amino acids 1-15 are removed, even more preferably at least the N-terminal amino acids 1-16 are removed, even more preferably at least the N-terminal amino acids 1-17 are removed.

SEQ ID NO: 3 represents the amino acid sequence:

KPTENNEDFNIVAVASNFATTDLDADRGKLPGKKLPLEVLKEMEANARKAGCTR
GCLICLSHIKCTPKMKKFIPGRCHTYEGDKESAQGGIGEAIVDIPEIPGFKDLEPMEQ
FIAQVDLCVDCTTGCLKGLANVQCSDLLKKWLPQRCATFASKIQGQVDKIKGAGG
DT

These amino acids represent amino acids 18-186 of the wild type gLuc encoded by the sequence SEQ ID NO: 17 (cf., Ruecker *et al.,* 2008).

Even more preferably, the gLuc has a sequence homology of at least 70 %, even more preferably at least 80 %, even more preferably at least 90 % to SEQ ID NO: 1 or may be, most preferably identical to SEQ ID NO: 3.

Alternatively, the *g*Luc is encoded by a nucleic acid having a sequence homology of at least 60 %, more preferably at least 70 %, even more preferably at least 80 %, even more preferably at least 90 %, even more preferably at least 95 % to SEQ ID NO: 4 or may be, most preferably identical to SEQ ID NO: 4.

SEQ ID NO: 4 represents the nucleic acid sequence:

AAGCCGACGGAGAACAACGAGGACTTCAACATTGTCGCCGTGGCGTCCAACTT
TGCCACCACCGATCTGGACGCCGACCGCGGCAAGCTGCCGGGCAAGAAGCTG
CCCCTGGAGGTCCTCAAGGAAATGGAGGCGAACGCCCGGAAGGCCGGCTGCA
CCCGCGGCTGCCTGATCTGCCTGTCCCACATCAAGTGCACGCCGAAGATGAAG
AAGTTCATTCCCGGCCGGTGCCACACCTACGAGGGCGACAAGGAGTCCGCGCA
GGGCGGCATTGGCGAGGCGATCGTGGACATCCCCGAGATTCCGGGCTTCAAGG
ACCTGGAGCCGATGGAGCAGTTCATCGCCCAGGTGGACCTGTGCGTGGACTGC
ACGACCGGCTGTCTGAAGGGCCTGGCCAATGTGCAGTGTTCCGACCTGCTCAA
GAAGTGGCTGCCCCAGCGCTGTGCCACCTTCGCCAGCAAGATCCAGGGCCAGG
TGGACAAGATTAAGGGCGCTGGCGGCGATACG

This sequence is codon optimized for *C. reinhardtii.* Therefore, it is different than the native *G. princeps* nucleotide sequence in its GC content.

In another preferred embodiment, the ORF further comprises a sequence encoding for a protease cleavage site, in particular wherein said sequence encoding for a protease cleavage site is located between the sequence encoding for the target polypeptide and the sequence encoding for the luciferase.

Alternatively or additionally, the sequence encoding for a protease cleavage site may also be located between the sequence encoding for the target polypeptide and the sequence encoding for the CAH1 secretion signal.

A protease cleavage site may be the cleavage site of any protease known in the art. As used herein, the terms "protease", "peptidase" and "proteolytic enzyme" may be understood interchangeably. Typically, the protease recognizes the sequence of a protease cleavage site and cleaves an amide bond between two amino acids. The protease may an endoprotease or an exoprotease. Preferably, the protease is an endoprotease. The protease is further preferably extensively specific to a sequence of at least three, more preferably of at lest four consecutive amino acids. Exemplarily, the protease may be a serine protease, a threonine protease, a cysteine protease, an aspartate protease a metalloprotease or a glutamic acid protease. Preferably, the protease cleavage site is the cleavage site of a specific protease such as, e.g., Factor Xa protease.

Exemplarily, a cleavage site may comprise a restriction site having the sequence IEGR (SEQ ID NO: 21).

Cleaving off unwanted parts of the initial polypeptide strand, the pure desired polypeptide strand remains and can be easily isolated and/or purified by any of the methods known in the art to do so. Several of such methods are exemplarily described in the context of isolating and purifying below and in the examples.

In the ORF of the expression vector of the present invention, the sequential order of the three encoding sequences (i.e., the sequence encoding for the CAH1 secretion signal (*cah1*), the sequence encoding for the luciferase (*luc*) and the sequence encoding for the target polypeptide (*tps*)) is not determined, but freely variable.

Therefore, *tps* may be localized upstream of *cah1* and *luc,* between *cah1* and *luc* or downstream of *cah1* and *luc.* Preferably, *tps* is either localized between *cah1* and *luc* or downstream of *cah1* and *luc.*

Nevertheless, the person skilled in the art will understand the mechanism of action of the CAH1 secretion signal, therefore, that, in eukaryotic host cells, said CAH1 secretion signal will most likely direct the polypeptide through the endoplasmic reticulum (ER) and the Golgi apparatus into vesicles for exocytosis. Therefore, most preferably, the CAH1 will be located at the N-terminus of the expressed polypeptide strand, i.e., N-terminally of the target polypeptide. Accordingly, in the expression vector, *cah1* may preferably be located upstream of *luc* and *tps.* Herein, "upstream" means that it is located towards the 5'-region of the sense strand coding for the other two encoding regions.

In a more preferred embodiment, the sequential arrangement of the encoding sequences in the ORF between the promoter and the terminator sequence is
a) 5' - X₁ *- cah1* - X₂ *- luc* - X₃ - *tps* - X₄ - 3';
b) 5'- X₁ *-cahl -* X₂ *- tps -* X₃ - *luc -* X₄ -3',
wherein:
*cah1* is the sequence encoding for the CAH1 secretion signal,
*luc* is the sequence encoding for the luciferase,
*tps* is the sequence encoding for the target polypeptide, and
either X₁, X₂, X₃ and X₄ can independently from another encode for a spacer sequence, a protease cleavage site, an intron and/or a purification tag or may be missing,
in particular wherein
X₁ is an intron,
X₂ encodes for a spacer sequence or is missing,
X₃ encodes for a protease cleavage, optionally further comprising a spacer sequence, and
X₄ encodes for a spacer sequence and/or a purification tag such as a polyhistidine (polyHIs) tag or is missing.

It will be understood by a person skilled in the art that X₁, X₂, X₃ and X₄ may independently from another may optionally also be combinations of one or more spacer sequence(s), one or more protease cleavage site(s), one or more intron(s) and/or one or more purification tag(s) or any combination of two or more thereof. Exemplarily, X₁, X₂, X₃ and/or X₄ may comprise one or more RBCS2 intron(s) (Eichler-Stahlberg *et al.,* 2009). The person skilled in the art will notice that an RBCS2 intron typically acts as an enhancer for gene transcription and is cleaved off the mRNA prior to being translated into a polypeptide strand and may also be within the nucleotide sequence coding for *luc, tps,* and/or *cah1.*

Alternatively, the sequential arrangement of the encoding sequences in the ORF between the promoter and the terminator sequence may be
c) 5'- X₁ *- cah1 -* X₂ - *tps*1 *-* X₃ - *luc -* X₄ - *tps*2 -3',
d) 5 '- X₁ - *cah1 -* - X₂ *- tps*1 - X₃ - *tps*2 - luc - X₄ -3',
e) 5'- X₁ *- cahl -* X₂ - *tps*1 - X₃ *- tps*2 - *luc* - X₄ - *tps*3 -3',
wherein:
*tps*1, *tps2* and *tps3* are the same or different polypeptide strands.

A highly preferable expression vector is the vector according to the examples.

The ORF of the expression vector of the present invention preferably further comprises a promoter. Preferably, the promoter controls the expression of the ORF. As used herein, the term "promoter" may be understood in the broadest sense as a region of a vector (most preferably a DNA strand) that facilitates the transcription of the ORF. The promoter may be located near the ORF, and will typically be located on the same strand and upstream. The promoter may be a homologous promoter or a heterologous promoter. Highly preferably, the promoter is a homologous promoter, i.e., a promoter of the species of the host cell. Should a heterologous promoter be used, it is preferably a promoter from a related species. Preferably, the promoter is a promoter active in eukaryotic origin, more preferably a promoter active in an autotrophic eukaryotic organism, even more preferably a promoter active in an photoautotrophic eukaryotic organism, even more preferably a promoter active in an plant, even more preferably a promoter active in a green alga, even more preferably a promoter active in a green microalga, even more preferably a promoter active in a *Chlorophyta,* even more preferably a promoter active in a *Chlorophycea,* even more preferably a promoter active in *Volvocales,* even more preferably a promoter active in *Chlamydomonadacea,* even more preferably a promoter active in *Chlamydomona,* in particular a promoter active in *Chlamydomonas reinhardtii.*

Highly preferably, the promoter is a promoter of eukaryotic origin, more preferably a promoter of an autotrophic eukaryotic organism, even more preferably a promoter of an photoautotrophic eukaryotic organism, even more preferably a promoter of an plant, even more preferably a promoter of a green alga, even more preferably a promoter of a green microalga, even more preferably a promoter of a *Chlorophyta,* even more preferably a promoter of a *Chlorophycea,* even more preferably a promoter of *Volvocales,* even more preferably a promoter of *Chlamydomonadacea,* even more preferably a promoter of *Chlamydomona,* in particular a promoter of *Chlamydomonas reinhardtii.*

The promoter may be a constitutive promoter or may be an inducible promoter. It may also be an inducible promoter that is combined with a second inducible promoter and thereby becomes a constitutively active promoter side. It may be a regulated or an extensively unregulated promoter that allows continual transcription of its associated gene. Highly preferably, the promoters are the ones as exemplarily shown in the examples.

In a preferred embodiment, the expression vector further comprises a heat shock protein promoter, preferably a heat shock protein 70A (HSP70A) promoter coupled to a ribulose-1,5-bisphosphate carboxylase/oxygenase small subunit (RBCS2) promoter. In this context both promoters act to create a constitutive promoter.

In a preferred embodiment, the expression vector further comprises an enhancer, preferably wherein said enhancer is a ribulose-1/5-bisphosphatase (RBCS2) intronic enhancer, in particular wherein said expression vector comprises the combination of an HSP70A - RBCS2 promoter and an RBCS2 intronic enhancer.

The term "enhancer" as used herein may be understood in the broadest sense as a short sequence that can be bound with proteins such as the trans-acting factors (much likely a set of transcription factors) to enhance transcription levels of the encoding sequence in the ORF of the present invention. Preferably, the RBCS2 enhancer is an RBCS2 intron. Herein, the HSP70A promoter may preferably be coupled to the RBCS2 promoter and the RBCS2 intron to create a constitutive promoter of different characteristic than any of the independent elements alone. Preferably, the enhancer controls the expression of the ORF.

In a preferred embodiment, the expression vector comprises a nucleic acid sequence having a sequence of at least 60 % sequence homology to SEQ ID NO: 5, in particular comprising a nucleic acid sequence encoding for a polypeptide having an amino acid sequence of at least 60 % sequence homology to SEQ ID NO: 6 or SEQ ID NO: 7.

The nucleotide sequence SEQ ID NO: 5 has the sequence:

atggcgcgtactggcgctctactcctggtcgcgctggcgcttgcgggctgcgcgcaggcttgcAGATCTAAGCCGA
CGGAGAACAACGAGGACTTCAACATTGTCGCCGTGGCGTCCAACTTTGCCACC
ACCGATCTGGACGCCGACCGCGGCAAGCTGCCGGGCAAGAAGCTGCCCCTGG
AGGTCCTCAAGGAAATGGAGGCGAACGCCCGGAAGGCCGGCTGCACCCGCGG
CTGCCTGATCTGCCTGTCCCACATCAAGTGCACGCCGAAGATGAAGAAGTTCA
TTCCCGGCCGGTGCCACACCTACGAGGGCGACAAGGAGTCCGCGCAGGGCGG
CATTGGCGAGGCGATCGTGGACATCCCCGAGATTCCGGGCTTCAAGGACCTGG
AGCCGATGGAGCAGTTCATCGCCCAGGTGGACCTGTGCGTGGACTGCACGACC
GGCTGTCTGAAGGGCCTGGCCAATGTGCAGTGTTCCGACCTGCTCAAGAAGTG
GCTGCCCCAGCGCTGTGCCACCTTCGCCAGCAAGATCCAGGGCCAGGTGGACA
AGATTAAGGGCGCTGGCGGCGATACG

This sequence represents a nucleic acid sequence encoding for a fusion polypeptide of a CAH1 secretion signal with *g*Luc (CAgLuc). The lowercase letters (nucleic acid positions 1-63) represent the CAH1 secretion signal-encoding sequence, the capital, underlined letters (nucleic acid positions 64-69), represent a restriction endonuclease recognition site-and the capital, non-underlined letters (nucleic acid positions 70-579) represent the modified N-terminal lacking gLuc-encoding sequence.

Even more preferably, the CAgLuc is encoded by a nucleic acid having a sequence with a sequence homology of at least 70 %, even more preferably at least 80 %, even more preferably at least 90 % to SEQ ID NO: 1 or may be, most preferably, identical to SEQ ID NO: 5.

As described above, the expression vector of the present invention comprises an ORF comprising sequences encoding for a CAH1 secretion signal, a target polypeptide and a luciferase.

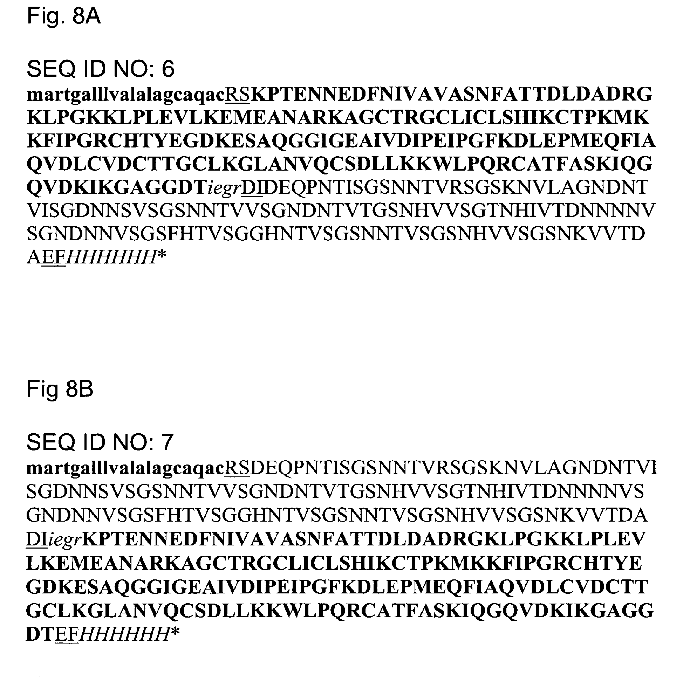

In one example, the expression vector may encode for a fusion polypeptide having a sequence with a sequence homology of at least 60 %, preferably of at least 70 %, even more preferably at least 80 %, even more preferably at least 90 % to SEQ ID NO: 1 or may be, most preferably, identical to SEQ ID NO: 6 representing a fusion polypeptide with C-terminal high value recombinant protein (HVRP) and a polyHis tag (CA-gLuc-HVRP). SEQ ID NO: 6 is also depicted in Figure 8A.

In another example, the expression vector may encode for a fusion polypeptide having a sequence with a sequence homology of at least 60 %, preferably of at least 70 %, even more preferably at least 80 %, even more preferably at least 90 % to SEQ ID NO: 1 or may be, most preferably, identical to SEQ ID NO: 7 representing a fusion polypeptide with HVRP located between the N-terminal CAH1 secretion signal (CA) and the C-terminal gLuc and a polyHis tag (CA-HVRP-gLuc). SEQ ID NO: 7 is also depicted in Figure 8B.

The person skilled in the art will notice that the HRVP region only represents an exemplary target polypeptide that can be replaced by any other target polypeptide known in the art.

As mentioned above, the encoding sequences of the expression vector of the present invention may be expressed in an extracellular, i.e., cell-free, expression system and/or in a host cell. Preferably, the vector is expressed in a host cell, most preferably in a host cell as defined below.

Therefore, a further aspect of the present invention relates to a host cell comprising the expression vector according to the present invention.

The host cell may be any cell suitable for expressing the encoding sequences of the expression vector of the present invention. It may be a plant cell (e.g., *Archueplastida,* glaucophyte algae (*Glaucophyta*), red algae (*Rhodophyta*), *Viriplantae,* green algae (e.g., *Chlorophyta, Streptophyta, Charales*)), land plants (e.g., *Embryophyta* (*Marchantiophyta, Bryophyta, Anthocerotophyta, Tracheophyta, Pteridophyta, Spermatophyta, Pinophyta, Cycadophyta, Ginkgophyta, Gnetophyta, Magnoliophyta*))*,* an animal cell (e.g., an insect cell, an crustacean cell, a vertebrate cell (e.g., a mammalian cell)), a bacterial cell (gram positive or gram negative), an archaea cell, a fungal cell (e.g., a yeast cell), or a hybrid of two or more thereof.

It will be apparent that, to obtain the host cell, the expression vector of the present invention has to be inserted into the cell. This insertion may also be understood as transformation of the cell. This can be performed by any means known in the art. Exemplarily, it may be inserted with or without transfecting agent(s) (e.g., lithium acetate, polyethylene imine (PEI), polyethylene glycol (PEG), fugene, LT-1, JetPEI, transfectamine, lipofectamine, UptiFectin, PromoFectin, GenePORTER, Hilymax, carbon nanofibres, glass bead agitation, carbon nanotubes cell-penetrating peptides (CPPs), protein transduction domains (PTDs), liposomes, DEAE-dextran, dendrimers), with or without electroporation, or with or without a gene gun, with or without optical transfection with or without gene electrotransfer, with or without impalefection, with or without magnetofection, and/or with or without magnet assisted transfection.

The expression vector may or may not be inserted into the genome of the host cell or may be plasmid. In a eukaryotic host cell, the expression vector will typically migrate into the nucleus and will be transcribed there.

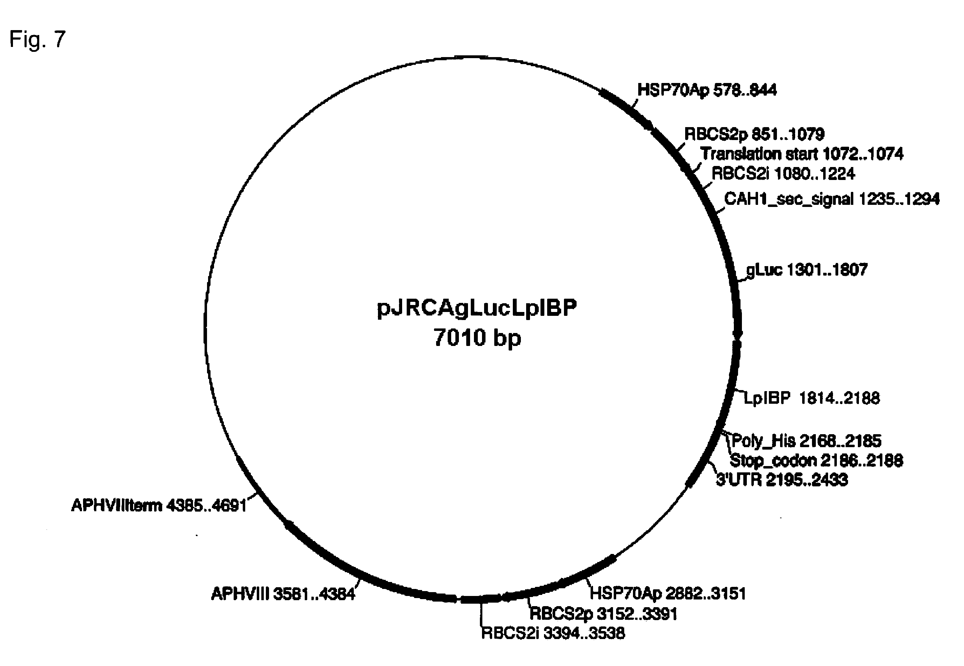

It will be apparent to one skilled in the art that the expression vector is also likely to contain any number of secondary expression regions that code for selectable markers such as antibiotic resistance or complementation to an auxotrophic phenotype. In a preferred example, a paromomycin antibiotic resistance selectable marker is present as depicted in Figure 7.

Optionally the host cells may be stabilized by freezing, freeze-drying or drying. Preferably, the cells are frozen. The cells may be frozen at -20°C, -80°C, on dry ice or in liquid nitrogen. Preferably, the cells may be frozen in a medium suitable to freeze yeast cells. This medium may contain glycerol. Preferably, the cells may be stabilized in aqueous buffers or water supplemented with more than 2 % (v/v), more than 5 % (v/v), more than 10 % (v/v), more than 20 % (v/v), more than 30 % (v/v), more than 40 % (v/v), or more than 50 % (v/v) glycerol. Exemplarily, yeast cells may be frozen in medium comprising 50 % (v/v) glycerol in water. As used throughout the invention, the abbreviation v/v refers to volume per volume. A batch of cells stabilized in a glycerol solution may be designated as glycerol stock. Alternatively, the yeast cells may be dried or freeze-dried, preferably by the addition of an emulsifier such as, e.g., a citric aci.e.er. Dried or freeze-dried cells may be stored at dry conditions at any temperature such as, e.g., at room temperature, ambient temperature, at -4°C, -20°C, -80°C, on dry ice or in liquid nitrogen. Most preferably, the cells are stabilized as exemplarily shown in the examples.

The stabilized cells may be stable for at least more than 1 day, preferably more than 5 days, more preferably more than 1 week, even more preferably more than 1 month, and even more preferably more than 1 year. As indicated above, "stable" means that the cell can be used to form a viable cell culture after the storage time.

In a preferred embodiment, the host cell is a eukaryotic cell, preferably an autotrophic eukaryotic cell, more preferably a green alga cell, even more preferably more preferably a green alga cell, in particular a green alga cell **characterized in that** it lacks a cell wall and/or it is a green alga cell from the division of *Chlorophyta,* preferably a *Chlorophycea* cell, more preferably a *Volvocales* cell, even more preferably a *Chlamydomonadacea* cell, even more preferably a *Chlamydomona* cell, in particular a *Chlamydomonas reinhardtii* cell.

In this context, an autotrophic eukaryotic cell is preferably a phototrophic cell, i.e. a cell carrying out photon capture to acquire energy. Preferably, phototrophic cell are obligatorily photosynthetic cells and, therefore, require light to generate energy. The phototrophic cell as used herein does preferably not need any organic energy source what allows the cultivation in a medium extensively free of sugars, amino acids and polypeptidic growth factors. However, cells may possess the ability to utilize other carbon sources. The cell may optionally lack a cell wall. Then, the cell may lack a cell wall genetically or the cell wall may be removed by any means known in the art (e.g., enzymatically). Optionally, the cell may also be a cell in a callus of plant cells.

As used herein, a green alga cell is preferably a microalga, i.e. an alga consisting of a single, two three or few cells having a size in the range of from 0.5 µm to 500 µm. The green alga cell may also be a diatom.

As used in the context of the present invention, a *Chlamydomona* cell may be any *Chlamydomona* cell. As already indicated above, the *Chlamydomona* cell is highly preferably *Chlamydomonas reinhardtii* (*C. reinhardtii*).

Alternatively, the *Chlamydomona* cell may also be *Chlamydomonas caudata* Wille (*C. caudata* Wille), *Chlamydomonas moewusii* (*C. moewusii), Chlamydomonas nivalis* (*C. nivalis*) or *Chlamydomonas dysosmos* (*C. dysosmos*) or others. Preferably, the *Chlamydomonas* species of the present invention lacks a cell wall, i.e. is extensively not surrounded by a solid cell wall mainly composed of proteins, cellulose and/or hemicellulose.

However, cells comprising a cell wall can also be used. Then, the polypeptide of the present invention accumulates in the space in between the cell membrane and the cell wall. The concept may also be applicable to other cell, in particular microalgae species given appropriate regulatory nucleic acids sequences.

In the view of the above, highly preferably, the present invention relates to a system for efficient recombinant protein production and detection from nuclear transgenes in *C. reinhardtii. Chlamydomonas reinhardtii* has been shown to be generally able to produce complex polypeptides comprising pharmaceutically relevant antibodies, human enzymes and other recombinant proteins (Specht *et al.,* 2010).

A further aspect of the present invention refers to a method for producing a polypeptide, comprising the following steps:
i) providing a cell according to the present invention;
ii) cultivating said cell of step i) in a culture broth under conditions allowing the production and secretion of said polypeptide; and
iii) isolating said polypeptide from said culture broth or the direct use of that broth for the target recombinant polypeptide's application.

Step i), i.e., the step of providing a cell, may be performable as described above.

Cultivating the cell will be performed by means suitable for the individual cell type. Herein, the terms "cultivating", "culturing" and "growing" may be understand interchangeably as any means for maintaining cell viability, preferably also allowing proliferation of the cells. Depending on the host cell type, cultivating may be cultivating adherently and/or in suspension. It may be cultivating in a cell culture flask or Erlenmeyer flask (e.g. plastic or glass, baffled or unbaffled, gassed or ungassed, shaken or unshaken), on a Petri dish (e.g. on a solid matrix such as, e.g., agarose), in an incubator (stirred or unstirred, gassed or ungassed, shaken or unshaken). The volume may be few milliliters, up to 50 ml, up to 100 ml, up to few liters, or even up to cubic meters. The temperature may be dependent on the host cell type. Typically, for most host cells, the incubation temperature will be in the range of from 0°C to 100°C, even more typically in the range of from 5°C to 50°C, even more typically in the range of from 10°C to 50°C, most typically in the range of from 15°C to 45°C.

Exemplarily, phototrophic cells, such as algae, are cultivated in a medium that is preferably extensively free of sugars, amino acids and polypeptidic growth factors. The person skilled in the art will notice that phototrophic cells need light of a suitable wavelength and intensity to survive and produce and secrete the polypeptide.

As indicated above, The CAH1 secretion signal may typically be removed during the secretion process. Therefore, optionally, depending on the individual sequence, the experimenter may obtain a protein comprising a target polypeptide and a luciferase without the CAH1 secretion signal. However, this depends on the sequence and a sequence that is not cleaved at this site may be chosen and, therefore, a polypeptide comprising the CAH1 secretion signal as well may be obtained.

As used herein, the term "isolating" may be understood in the broadest sense as obtaining the polypeptide from the culture broth. Isolation means that the content of the polypeptide in a sample is increased and unwanted elements from the culture broth are substantially removed. Hereby, typically, the cells are separated from the medium surrounding the cells by any means in the art such as, e.g., by centrifugation, by filtration, by crossflow filtration or by chromatography (e.g., affinity, size exclusion, ion-exchange chromatography). Exemplarily, the method of the present invention enables continuous polypeptide production through simple media filtration in a perfusion style cultivation strategy.

Alternatively or additionally, the polypeptide may be isolated further, depending on its specific chemical nature, e.g., by chromatographic methods (e.g., phase chromatography, ion-exchange chromatography, reverse phase chromatography, size exclusion chromatography, high performance liquid chromatography (HPLC), ultrahigh pressure liquid chromatography (UPLC), fast protein chromatography (FPLC)), by electrophoresis, capillary electrophoresis (CE), by distillation, by precipitation (e.g., salting is or salting out, solvent extraction), etc. Herein, the concentration is increased further.

Steps ii) and iii) may be conducted subsequently or simultaneously.

The method of the present invention may also be designated as efficient and codon optimized polypeptide expression, secretion and detection system (ECP-PESED), preferably for green microalgae, in particular for the green microalga *Chlamydomonas reinhardtii.*

In a preferred embodiment, the steps ii) and iii) are conducted simultaneously, in particular wherein cultivating is continuous cultivating.

Because the polypeptide is preferably obtained from the medium around the cells, the cells are preferably cultivated at the same time when the polypeptide is obtained and isolated.

Preferably, the cells do not have to be lysed to obtain the polypeptide. Therefore, preferably, the cells do not have to be subjected to any of the steps of mechanical force (e.g., by homogenization (e.g., by a Potter or a Downs homogenizer), by a cell press such as, e.g., a French press), by detergents, by sonication, or by lytic phages.

In a preferred embodiment, the method further comprises at least one of the steps selected from the group consisting of:
iv) detecting bioluminescence from the luciferase;
v) enzymatically cleaving the target polypeptide from the luciferase; and
vi) purifying said target polypeptide.

As already described above, detecting above may be performed by visualizing and/or quantifying by measuring chemiluminescence or by a photon counting camera and serve as a quantitative or semi-quantitative method of identifying relative polypeptide abundance because the light emission occurs on a per-unit basis, thus, samples with more units of gLuc produce more light with an extensively linear correlation. Exemplarily, colonies of the host cell expressing *g*Luc may be identified in their culture medium (e.g., on Petri dishes) through a simple coelenterazine spray followed by luminescence imaging. This is further shown in detail in the examples.

As used herein, "enzymatically cleaving" means that the polypeptide strand is cleaved by means of an enzyme, most particularly a protease, preferably a protease as defined above.

As used in the context of the present invention, "purifying" means that the concentration of the polypeptide is increased up to a degree that it encounters for at least 30 % (w/w), at least 50 % (w/w), at least 60 % (w/w), at least 70 % (w/w), at least 80 % (w/w), at least 90 % (w/w), at least at least 95 % (w/w) or at least 98 % (w/w) of the total protein content.

Herein, the step of purifying the target polypeptide may be conducted by any means of known in the art such as, e.g., by precipitation (e.g., by solvent extraction with an organic solvent (e.g., acetone or diethyl ether), salting in, salting out), by chromatographic methods (e.g., phase chromatography, ion-exchange chromatography, reverse phase chromatography, size exclusion chromatography, high performance liquid chromatography (HPLC), ultrahigh pressure liquid chromatography (UPLC), fast protein chromatography (FPLC)), by electrophoresis, capillary electrophoresis (CE), or by distillation.

The person skilled in the art will notice that the polypeptide may further be subjected to one or more posttranslational and/or chemical modifications. As used herein, the posttranslational and/or chemical modifications may be introduced by the user of the method of the present invention on purpose or without intention or may occur naturally in the cell or in the medium.

Posttranslational modifications are well-known in the art and may comprise but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, nitration, nitrosylation, disulfide bond formation, pyroglutamate formation, amino acid addition, cofactor addition (e.g., biotinylation, heme addition) and complexation of metal ions, non-metal ions, peptides or small molecules and addition of iron-sulfide clusters. Further, co-factors, such as, e.g., ATP, ADP, NAD+, NADH+H⁺, NADP⁺, NADPH+H⁺, metal ions, cations, anions, lipids may be bound to the polypeptide, irrespective on their biological impact. Moreover, the protein may be elongated by one or more amino acids.

Chemical modifications include, but are not limited to the conjugation of one or more protein tag(s) (e.g., dihydrofolate, polyhistidine tag(s) ((poly)His tag(s)), streptavidin), one or more binding moiety/moieties comprising, e.g., biotin, methotrexate, glycocorticoid(s), one or more active ester(s) (e.g., N-hydroxysuccinimidyl (NHS) ester(s)), one or more isothiocyanate(s), one or more maleimide(s), one or more glutaraldehyde derivative(s), one or more carbodiimide derivative(s) or combinations thereof, one or more insoluble and/or soluble polymer(s) (e.g., polyethylene glycol (PEG), hydroxypropyl methacrylate (HPMA), polyethylene imine (PEI)), one or more antibody/antibodies or derivative(s) thereof, micro- or nanobeads (e.g., functionalized silica beads, polysaccharide-based beads), polymersomes, liposomes, one or more linker or spacer molecule(s) such as, e.g., peptide linker(a), a polyethylene glycol (PEG) linker(s), saccharide linker(s), fatty acid linker(s), alkyl linker(s), antibody linker(s), or a combination of two or more thereof.

Furthermore, the term "polypeptide" as used in the context of the present invention may also include pharmaceutical acceptable salts thereof.

A highly preferable method is the method according to the examples. It will be understood that the specifications in the context of the expression vector according to the present invention described above also apply to the method according to the present invention.

In a further aspect, the present invention refers to the use of a cell according to the present invention for producing a polypeptide.

It will be understood that the specifications in the context of the method and/or the expression vector according to the present invention described above also apply to the use according to the present invention.

The invention is further explained by the following example and figures, which are intended to illustrate, but not limit the scope of the present invention.

### Figures

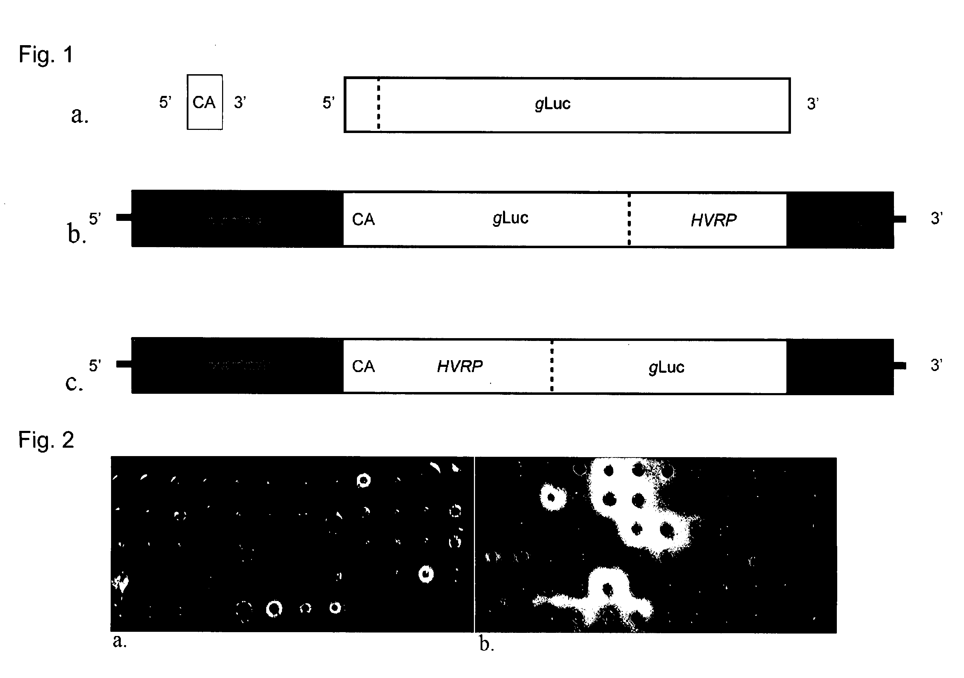

**Figure 1** shows the genetic elements and example orientations. In a., CA represents the nucleotide sequence coding for the first 21 amino acids of the CAH1 secretion signal gene of *Chlamydomonas reinhardtii* (Fukuzawa *et al.,* 1990). gLuc represents the nucleotide sequence coding for amino acids 18-186 of the *Gaussia princeps* luciferase. b. shows a representative vector with a CA sequence fusion with gLuc sequence to place a high value recombinant protein (HVRP) in a C-terminal position. c. shows the N-terminal placement of gene of interest. All vectors require a gene promoter in the 5' position, 3' un-translated region terminator sequence, and will likely also employ intronic sequences as transcription enhancer elements.
**Figure 2** shows the results of a bioluminescence assay of transformant colonies at plate level. **a.** Without addition of the CA sequence, intracellular gene expression occurs resulting in bioluminescence signal when 0.1 mM coelenterazine in ethanol and assay buffer is incubated on colonies. **b.** Extracellular secreted protein is clearly visible from constructs expressing both normal gLuc secretion signal and CA construct. Pictured here, CA mutants are assessed for their relative protein production by incubating colony plates with 0.01 mM coelenterazine in assay buffer.

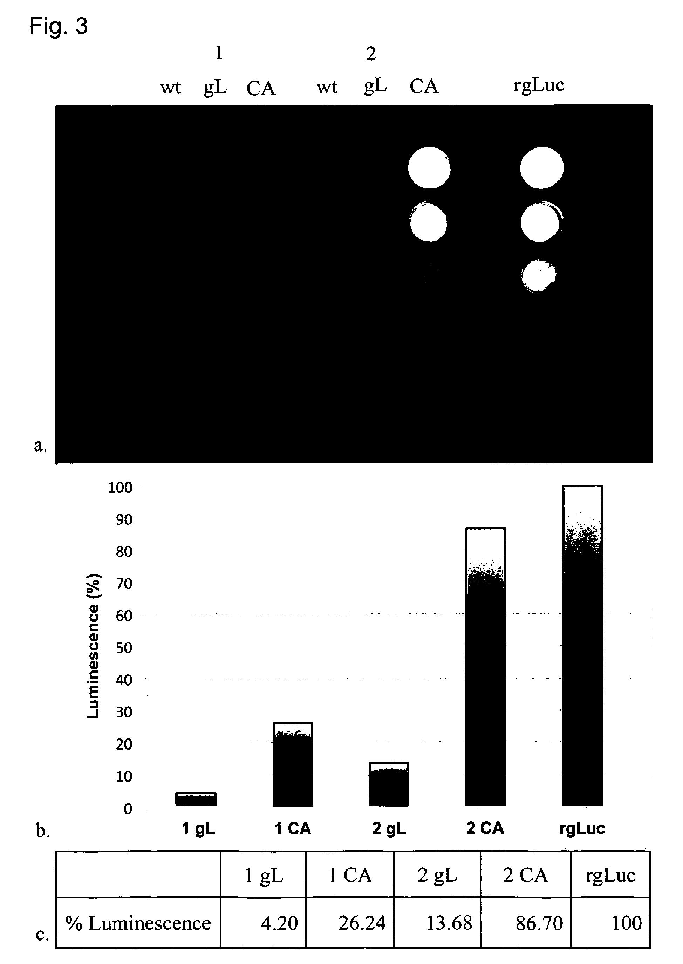

**Figure 3** shows a semi-quantitative luminescence signal analysis of media samples harvested from stationary phase cultures. **a.** Two different *Chlamydomonas reinhardtii* strains CC 1883 (1) as well as UVM4 (2) (Ruecker *et al.,* 2008) are depicted. Wild-type negative controls (wt), native *Gaussia* luciferase secretion signal (gL), and the CA gLuc fusion (CA) mutant media samples are shown in dilution assay from pure media (top) to 1:10 dilution (bottom) compared to a commercial standard from 0.01-0.001 mg/ml (rgLuc). **b.** Graphic representation of relative luminescence signals. c. Percentages relative to standard signal for each mutant.

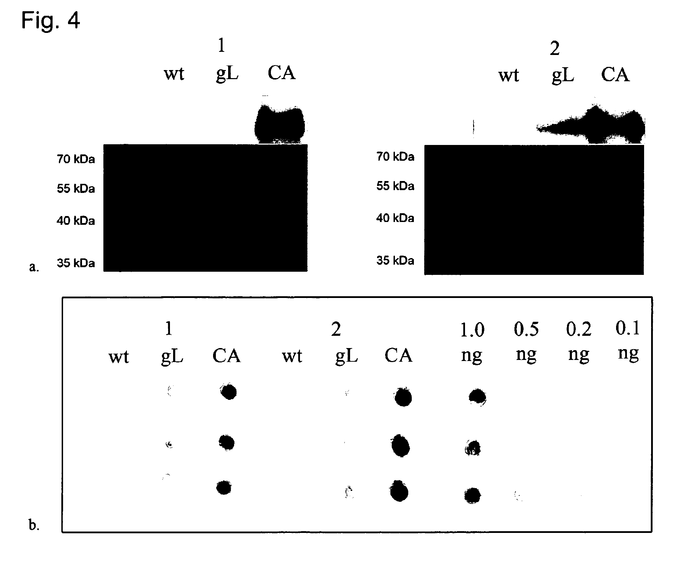

**Figure 4** shows protein quantification through SDS PAGE and Western blotting analysis with anti-*g*Luc antibody. **a.** Media from CC 1883 (1) and UVM4 (2) mutants produce a signal just below 55 kDa. Signals are present in both mutants of each strain and demonstrate stronger signal from CA fusion construct expressing mutant samples. **b.** Quantification of protein signals by dot blot analysis. Both CA fusion mutants clearly demonstrate greater protein abundance accumulation from than the native gLuc secretion signal. Both CA constructs demonstrate above 1 ng/µl (1 mg/L) recombinant protein accumulation in media outside of the cell.

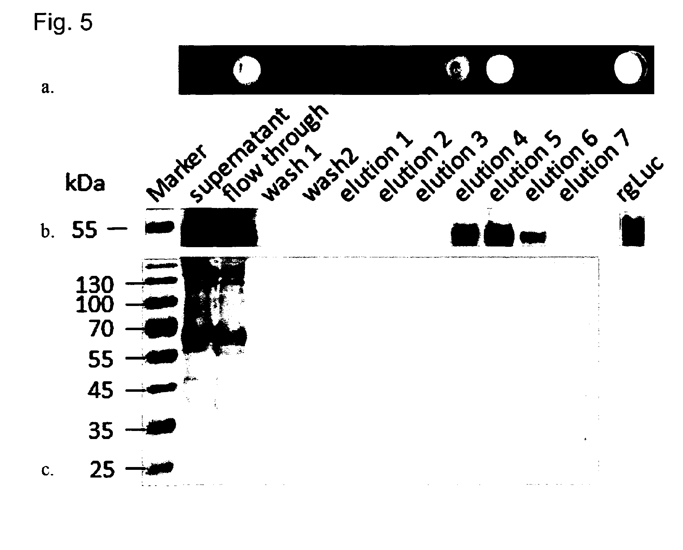

**Figure 5** shows the results of nickel affinity chromatography of target HVRP from UVM4 CA mutant culture media. **a.** Relative luminescence of Ni affinity fractions. **b.** Western blot analysis of samples taken from Ni affinity purification, in elution fractions a clear band is visible slightly under 55 kDa. c. Samples subjected to SDS PAGE, no clear protein band is visible, likely do to the small volume of culture media used

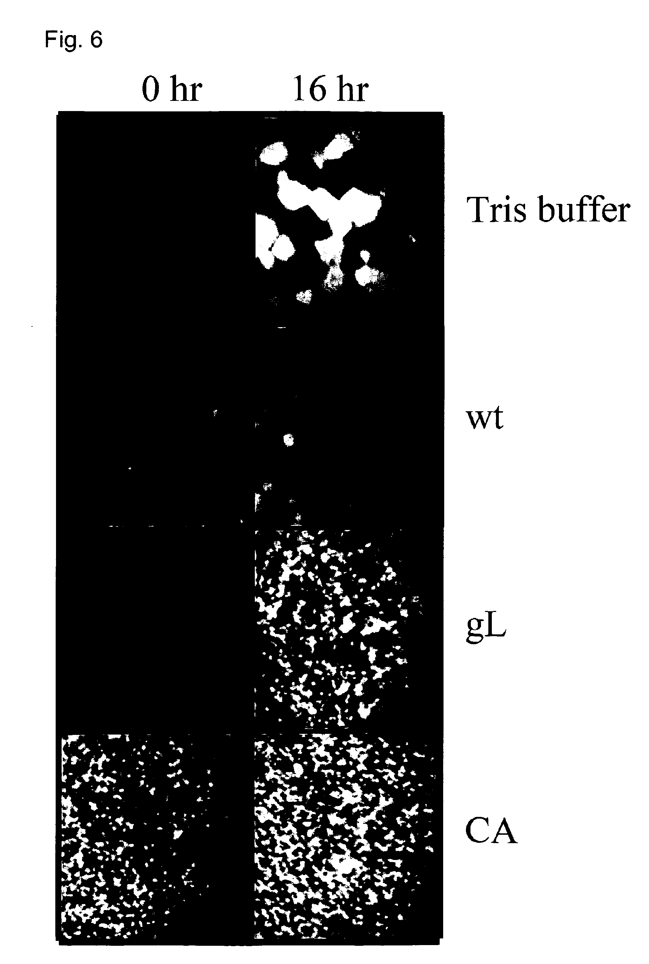

**Figure 6** shows an ice recrystallization inhibition (IRI) assay of pure culture media samples isolated from stationary phase UVM4 cultures. No IRI is visible in Tris buffer or wild-type media sample, a small amount of activity is visible in gL mutants and strong IRI is visible in CA samples.
**Figure 7** shows the vector diagram of the pJR**CA***g*Luc*Lp*IBP (SEQ ID NO: 16) vector including restriction endonuclease sites and regulatory elements of interest. pJR**n**s*g*Luc*Lp*IBP (SEQ ID NO: 14) and pJR*g*Luc*Lp*IBP (SEQ ID NO: 15) differ only between the *Nde*I and *Bgl*II restriction endonuclease sites. In this vector diagram, "HSP70A-RBCS2" at position 578-1079 represents a constitutive fusion promoter, "RBCS2i" at position 1075-1224 represents the RBCS2 intron, translational enhancer element, "CAHI_sec_signal" at position 1235-1294 represents the CAH1 secretion signal, "*g*Luc" at position 1301-1807 represents the codon optimized *Gaussia princeps* luciferase with native secretion signal removed, "*Lp*IBP" at position 1814-2188 represents the codon optimized *Lolium perenne* ice binding protein with hexaHIS tag and "3'UTR" at position 2195-2433 represents an untranslated region, terminal mRNA regulatory region.
**Figure 8** exemplarily demonstrates two orientations of the fusion polypeptide between the CAH1 secretion signal (depicted in bold lowercase letters), *g*Luc (depicted in capital bold letters), the anti-freeze-peptide HVRP (capital regular letters). Figure 8A shows the orientation CAH1 secretion signal-*g*Luc-HVRP. Figure 8B shows the orientation CAH1 secretion signal-HVRP-*g*Luc. Both polypeptides further comprise a cleavage site (lowercase italics), amino acids encoded by restriction enzyme sites for *Bgl*II and *Eco*RV (underlined) and a polyHIS tag (capital italics).

### Examples

### Materials and Methods

### Vector construction

The sequence of plasmid pHsp70A/RbcS2-c*g*Luc (Fuhrmann *et al.,* 2004; Heitzer *et al.,* 2007) between the restriction endonuclease sites *Sac*I and *Kpn*I was used as a template to design a new vector for nuclear transformation and transgene expression in *C. reinhardtii.* This sequence was modified to contain a codon optimized version of the *Gaussia princeps* luciferase (gLuc) marker with C-terminal *Lolium perenne* ice binding protein (*Lp*IBP) and hexa-histidine (hexaHIS) tags. Wild type gLuc may be encoded by the amino acid sequence of SEQ ID NO: 17. Wild type *Lp*IBP may be encoded by the amino acid sequence of SEQ ID NO: 19. Unique restriction digest sites were also inserted between each regulatory and gene element. The sequence of *Bgl*II was inserted in the coding region immediately after the gLuc predicted N-terminal secretion signal determined by SignalP server. The entire gene expression cassette was synthesized by GeneArt (Life Technologies) through oligonucleotide annealing. This 1.8 kb fragment was subcloned into vector pJR389 between restriction endonuclease sites *Spe*I and *Psh*AI to incorporate its paromomycin resistance cassette. DNA was digested with FastDigest® restriction endonucleases, samples were run on a 2 % agarose gel at 120 V for 35 min were visualized on a BioDocAnalyze Live camera (Biometra) after staining with SYBR® Safe DNA Gel Stain (Life technologies). Linear fragments were excised from the gel and purified with peqGOLD Gel Extraction Kit (Peqlab) following manufacturers protocols and then ligated with Quick T4 DNA Ligase (NEB) and transformed into Dh5α *Escherichia coli* cells by heat shock. The resulting vector is called pJR*g*Luc*LpIBP* and encodes resistance to the antibiotic paromomycin. From this vector, two further vectors were created.

pJR**CA***g*Luc*LpIBP* contains the *Chlamydomonas reinhardtii* carbonic anhydrase 1 (CAH1) secretion signal (CA) (Fukuzawa *et al.,* 1990) in place of the predicted secretion signal of the gLuc. Wild type full length CAH1 is typically encoded by the sequence of SEQ ID NO: 18. To create this vector, the entire 1.1 kb sequence of CAH1 was synthesized as above and the N-terminal targeting sequence was subsequently amplified by PCR using the primers FW_{*Nde*I} 5'-aattcatatgGCGCGTACTGGCGCTCT-3' (SEQ ID NO: 8), RV_{*Bg*/II} 5'-aattagatctAGCCTGCGCGCAGC-3' (SEQ ID NO: 9). PCR was conducted with Platinum^{®}Pfx DNA polymerase with recommended reagents. PCR was cycled with: 95 °C for 2:00 min, 35 cycles of 95 °C for 30 s, 68 °C for 30 s, 72 °C for 15 s, followed by 72 °C for 8:00 min. Reactions resulted in a single visible band of approximately 60 bp that was processed as above. This fragment, as well as the vector pJR*g*Luc*LpIBP*, were digested with *Nde*I and *Bg*/II run on a 2 % agarose gel and processed through to ligation as above. Positive transformants were screened for appropriate ligations by PCR and isolated plasmids were confirmed by sequencing. The resulting vector, pJRCA*g*Luc*LpIBP*, harbors the CAH1 N-terminal targeting secretion signal in place of the gLuc secretion signal. Therefore, the vector may comprise a sequence encoding for a fusion polypeptide comprising the CAH1 secretion signal, gLuc and the antifreeze peptide *Lp*IBP (CA*g*Luc*Lp*AFP) having a sequence of SEQ ID NO: 20.

A third vector was designed to omit the predicted secretion sequence of the gLuc gene altogether. This was achieved through amplification of the codon optimized gLuc sequence by PCR using the vector pJR*g*Luc*LpIBP* as a template with the primers FW_{*Nde*I} *5'-*aattcatatgAAGCCGACGGAGAACAACGA-3' (SEQ ID NO: 10), RV_{*Eco*RV} 5'-aattgatatcCGTATCGCCGCCAGCGC-3' (SEQ ID NO: 11). PCR was performed as above with an extension time of 30 s. The amplified DNA fragment was then subcloned into pJR*g*Luc*LpIBP* in place of the gLuc fragment as above between the restriction endonuclease sites *Nde*I and *EcoR*V resulting in the vector pJR**ns***g*Luc*LpIBP*. All vector sequences were confirmed through sequencing by SeqLab (Göttingen, Germany).

### Chlamydomonas reinhardtii strains, cultivation, and transformation

Wild type *Chlamydomonas reinhardtii* CC 1883 (*Chlamydomonas* center database) and strain UVM412 were both cultivated for this work. Unless otherwise indicated, these strains were cultivated in TAP or TAP_{agar} media under continuous light regimes of 150 µEm⁻²s⁻¹. Growth in HSM liquid media was conducted with stirring in 400 mL cultures with 3 % purged CO₂ at 450 pEm⁻²s⁻¹. Transformations were conducted by glass bead method as previously described (Kindle, 1990). Prior to transformation, vectors were linearized with *Spe*I and *Kpn*I restriction endonucleases and purified using peqGOLD Gel Extraction Kit (Peqlab) following manufacturer's protocols. Approximately 3 µg of vector DNA was used in each transformation. Transformations were plated on TAP_{agar} with 10 mg/L paromomycin under continuous light for 7 days until antibiotic resistant colonies were visible. Primary transformants were picked from plate and transferred to a 15 cm plate of the same media so that each colony was placed in an orientation allowing 96 independent colonies/plate. Replicate plate generation was performed simply with common 96 element stamping tools. Generally, colonies were allowed to grow for at least 1 week prior to assay or replicate plating to allow appropriate biomass accumulation.

### Bioluminescence assay

Assay of expression of gLuc marker protein was conducted using buffers and coelenterazine at concentrations previously described (Shao *et al.,* 2008). To conduct TAP_{agar} plate level luminescence assessment, three replicate plates of both CC 1883 and UVM4 mutant libraries for each vector were generated. Each plate was left in the dark for at least 5 minutes to prevent colony autofluorescence interference. Dark adapted colonies were covered with 0.01 mM coelenterazine in assay buffer (Shao *et al.,* 2008) and visualized for 1 minute in the FUSION FX7™ bioluminescence imaging camera (Peqlab) with native software (Figure 2b). Intracellular expression could be similarly visualized using 0.1 mM coelenterazine solution with 5 min exposure (Figure 2a). Images were compared for each replicate plate to determine colony luminescence patterns. 4 colonies demonstrating consistent and robust luminescence were isolated from libraries and cultivated separately. After 1 week of growth, selected mutant lines were screened as above for luminescence signal on a single plate. The mutant demonstrating the most robust luminescence signal was selected for further analysis. In total, one mutant strain of both UVM4 and CC 1883 containing each vector were isolated for further analysis.

### Quantification of recombinant protein from media

Liquid cultures of each mutant were cultivated in 100 mL TAP media and allowed to reach the late logarithmic phase (9.0 x 10⁶ cell/mL CC 1883, 2.3 x 10⁷ cells/mL UVM4). 100 mL of culture was centrifuged at 7500 rpm and supernatant decanted. The supernatant (culture media) was then run through a 0.2 µm syringe filter to remove cell debris and frozen in liquid N₂. Opaque 96 well microtiter plates were set in a dilution series with a standard commercially available *Gaussia* luciferase (PJK Shop, Kleinblittersdorf, Germany). The recombinant luciferase was resuspended to a concentration of 1 mg/mL in distilled water and then diluted to 0.01 mg/mL in TAP media prior to assay. Dilutions in plate were conducted with luciferase assay buffer. Isolated culture media was thawed on ice and pipetted in dilution series in the same manner as recombinant standard. 0.01 mM coelenterazine in assay buffer was pipetted into each lane using a multi channel pipette and samples were visualized in the FusionFX camera with 30 s exposure times. Relative luminescence was quantified using Biold software (Vilber Lourmat). Recombinant gLuc of 0.01 mg/mL (0.49 µM) was set as 100 %. All assays were conducted a minimum of three times.

### Confirmation of vector insertion PCR

Mutant colonies demonstrating both growth on paromomycin containing media and bioluminescence signals were assumed to contain genes of interest, however, a conformational PCR was also performed. DNA was isolated from mutants of interest via the Chelex method and 1 µl of resulting supernatant was used in PCR with the primers FW 5'-AAGCCGACGGAGAACAACGA-3' (SEQ ID NO: 12), RV 5'-GGCGTCGGTCACCACCT-3' (SEQ ID NO: 13). 1 µl (approximately 5 ng) of pJR*g*Luc*LpIBP* vector DNA was used as a positive control. PCR was performed as above with 45 s extension time.

### SDS PAGE and Western blot analysis from culture media

50 mL of culture media was lyophilized and resuspended to a 10fold concentration in distilled water. Samples were centrifuged for 3:00 min at 5000 g to remove insoluble fraction. 7µL of concentrated media was supplemented with 2x SDS-Sample buffer prior to loading onto sodium dodecyl sulphate polyacrylamide gel for electrophoresis (SDS PAGE). Samples were separated by Tris-Glycine-SDS-PAGE and blotted on nitrocellulose membranes (GE Healthcare) (Figure 4a). Immunodetection of gLuc was performed with an anti-gLuc antibody (NEB, MA, USA) as described by the supplier using enhanced chemiluminescence (ECL; GE Healthcare). Recombinant gLuc (PJK Shop) served as positive control.

Media was subjected to dot blot analysis for recombinant protein quantification (Figure 4b). 1 µl of pure culture media isolated as above was dotted in triplicate on a nitrocellulose membrane and let to dry. Standards of recombinant *Gaussia* luciferase produced in *Kluyveromyces lactis* (Generously supplied by Avidity, CO, USA) were diluted and added to the membrane to protein quantities shown.

### Nickel affinity chromatography

50 mL of culture media was lyophilized and resuspended in 5 mL affinity buffer (50 mM sodium phosphate buffer pH 8, no imidazole). An insoluble fraction was left after centrifugation and 0.8 mL of Ni resin was mixed with 4 mL of media solution under slow rotation at 4 °C for 60 min. Wash and elution buffers contain 300 mM NaCl. Two washes of 10 mL containing 0 mM imidazole were performed. Elution fractions of 1 mL contained imidazole concentrations of 10, 20, 50, 100, 150, 200, and 250 mM respectively. Samples were precipitated via methanol protein precipitation prior to preparation for SDS PAGE analysis as above.

### SPLAT ice recrystallization inhibition assay (IRI)

SPLAT assays were conducted as previously described (Knight *et al.,* 1988). Briefly, 10 µl of culture media was dropped onto microscope slides and snap frozen at -50 °C. Frozen crystals were imaged between polarized filters. Slides were held at -4 °C overnight for approximately 16 hours in an ethylene glycol bath and imaged again. Tris buffer alone was used as a negative control in addition to media isolated from the parental *Chlamydomonas reinhardtii* strain.

### Results

### Culture growth conditions

All mutant cultures demonstrate normal growth compared to parental strains. The average culture density at stationary phase for UVM4 is approximately 2.2x10⁶ cells/mL and 9.1x10⁶ cells/mL for CC 1883. This difference in culture density is related to the cell size of each strain, with CC 1883 demonstrating large round cells around 10 µm in diameter compared to 4-6 µm for UVM4 in TAP media. No significant difference is observed in culture biomass accumulation between the two strains.

### Bioluminescence

No luminescence signal is visible from parental strains of *C. reinhardtii,* either in media samples or at plate level. Replacing the predicted secretion signal of the *Gaussia* luciferase with the CA N-terminal targeting sequence dramatically increases protein production and localization into surrounding culture media in both strains of *Chlamydomonas reinhardtii* consistently by 84 % compared to the native gLuc secretion signal (Figure 3a-c).

### Recombinant protein quantification

UVM4 strains expressing the CA-gLuc fusion demonstrated recombinant protein production greater than 1 mg/L (Figure 4b). UVM4 for both constructs expresses recombinant proteins 70 % greater than CC 1883. Recombinant protein concentration is dependent on cell density, however, the greater cell density of UVM4 compared to 1883 does not entirely account for increase protein accumulation abundance. For biotechnological interest, UVM4 far outperforms CC 1883 in batch production per unit time accumulating both more cells/mL and more recombinant protein /L.

### Intracellular recombinant protein expression

Luminescence can be analyzed intracellularly, however, with higher concentrations of coelenterazine. Recombinant protein abundances from mutants expressing pJR**n**s*g*Luc*LpIBP* subjected to plate level luminescence screening could be identified based on relative intracellular luminescence. After centrifugation and media purification, no luciferase signal can be seen from culture media of these mutants.

### SDS and Western blot analysis

Recombinant protein products cannot be seen as independent bands in SDS PAGE either intracellularly or in media samples. The commercially available antibody for *Gaussia* luciferase is able to detect recombinant protein accumulation in all mutants observed in Western blot analysis (Figure 4a). *Lp*IBP is a 13 kDa protein that is known to run at a height of 25 kDa when produced in E. coli8 and at either 30 or 35 kDa from yeast10 due to glycosylation. Higher apparent molecular weight has been postulated to be the result of a biased amino acid sequence and is common in many ice binding proteins. The recombinant gLuc has a predicted molecular weight of 18.27 kDa. The gLuc-LpIBP fusion protein is predicted to be 31.25 kDa, however, due to the abherent gel migration of the LpIBP and glycosylation, is visible as a single band slightly less than 55 kDa (Figure 4a and 5b).

### Ni affinity chromatography

Nickel affinity chromatography is possible from the hexaHIS tag on the C-terminus of the *Lp*IBP fusion protein. From 50 mL of culture no band is visible on SDS PAGE, however, Western blotting demonstrates a single band of slightly less than 55 kDa (Figure 5b-c). Luminescence patterns from coelenterazine bioluminescence assay of Ni-affinity fractions match Western blotting signal intensities (Figure 5a-b).

### Ice binding activity

Ice recrystallization inhibition is observed from pure media in stationary phase CA cultures (Figure 6). The relative increase in protein abundance from the engineered CA-gLuc construct has allowed the LpIBP to surpass its dilution threshold of 0.055 µM14. gL secretion cultures exhibit a reduced abundance of recombinant protein accumulation (Figure 3, 4, and 6). gLuc cultures demonstrate weak IRI activity which corresponds to protein abundances (Figures 4b and 6). No inherent IRI is observed in wild-type strains (Figure 6).

### General findings

By working in concert with inherent signaling mechanisms, the fusion peptide facilitates increased transgene expression and the production of fusion high-value recombinant proteins by 84 % compared to the inherent secretion signal of the luciferase alone. This construct provides a mechanism to produce numerous high value recombinant protein targets from *Chlamydomonas reinhardtii* by secretion, into culture medium. Further, CAH1 has been identified as being superior of the other secretion signals such as, e.g. the arylsulfatase 2 (ARS2) secretion signal as used previously (Eichler-Stahlberg *et al.,* 2009).

The technology presented demonstrates the potential for the production of target polypeptides either for functional study or for applied roles. Other applications may include, but are not limited to the production of monoclonal antibodies, production of biomass degrading enzymes, production of bioremediation enzymes and consequent use of algal cultures as bio-based effluent filters, animal, human and medically relevant enzymes, and any other target polypeptide.

### References

Cao, M., Fu, Y., Guo, Y. & Pan, J. Chlamydomonas (Chlorophyceae) colony PCR. Protoplasma 235, 107-110, doi:10.1007/s00709-009-0036-9 (2009).
Eichler-Stahlberg, A., Weisheit, W., Ruecker, O. & Heitzer, M. Strategies to facilitate transgene expression in Chlamydomonas reinhardtii. Planta 229, 873-883, doi:10.1007/s00425-008-0879-x (2009).
Fuhrmann, M., Hausherr, A., Ferbitz, L., Schöd1, T., Heitzer, M. & Hegemann, P. Monitoring dynamic expression of nuclear genes in Chlamydomonas reinhardtii by using a synthetic luciferase reporter gene. Plant Molecular Biology 55, 869-881, doi:10.1007/s11103-004-2150-6 (2004).
Fukuzawa, H., Fujiwara, S., Yamamoto, Y., Dionisio-Sese, M. L. & Miyachi, S. cDNA cloning, sequence, and expression of carbonic anhydrase in Chlamydomonas reinhardtii: regulation by environmental CO2 concentration. Proceedings of the National Academy of Sciences 87, 4383-4387 (1990).
Heitzer, M. & Zschoernig, B. Construction of modular tandem expression vectors for the green alga Chlamydomonas reinhardtii using the Cre/lox-system. BioTechniques 43, 324-332 (2007).
Kindle, K. L. High-frequency nuclear transformation of Chlamydomonas reinhardtii. Proceedings of the National Academy of Sciences 87, 1228-1232 (1990).
Knight, C. A., Hallett, J. & DeVries, A. L. Solute effects on ice recrystallization: An assessment technique. Cryobiology 25, 55-60, doi:10.1016/0011-2240(88)90020-x (1988).
Lauersen, K. J., Brown, A., Middleton, A., Davies, P. L. & Walker, V. K. Expression and characterization of an antifreeze protein from the perennial rye grass, Lolium perenne. Cryobiology 62, 194-201, doi:10.1016/j.cryobiol.2011.03.003 (2011).
Neupert, J., Karcher, D. & Bock, R. Generation of Chlamydomonas strains that efficiently express nuclear transgenes. The Plant Journal 57, 1140-1150, doi:10.1111/j.1365-313X.2008.03746.x (2009).
Pudney, P. D. A., Buckley, S. L., Sidebottom, C. M., Twigg, S. N., Sevilla, M. P., Holt, C. B., Roper, D., Telford, J. H., McArthur, A. J. & Lillford, P. J. The physico-chemical characterization of a boiling stable antifreeze protein from a perennial grass (Lolium perenne). Archives of Biochemistry and Biophysics 410, 238-245, doi:10.1016/s0003-9861(02)00697-5 (2003).
Ruecker, O., Zillner, K., Groebner-Ferreira, R. & Heitzer, M. Gaussia-luciferase as a sensitive reporter gene for monitoring promoter activity in the nucleus of the green alga Chlamydomonas reinhardtii. Molecular Genetics and Genomics 280, 153-162, doi:10.1007/s00438-008-0352-3 (2008).
Shao, N. & Bock, R. A codon-optimized luciferase from Gaussia princeps facilitates the in vivo monitoring of gene expression in the model alga Chlamydomonas reinhardtii. Current Genetics 53, 381-388, doi: 10.1007/s00294-008-0189-7 (2008).
Specht, E., Miyake-Stoner, S. & Mayfield, S. Micro-algae come of age as a platform for recombinant protein production. Biotechnology Letters 32, 1373-1383, doi:10.1007/s10529-010-0326-5 (2010).
Yu, S. O., Brown, A., Middleton, A. J., Tomczak, M. M., Walker, V. K. & Davies, P. L. Ice restructuring inhibition activities in antifreeze proteins with distinct differences in thermal hysteresis. Vol. 61 327-334 (Elsevier, 2010).

## Claims

1. An expression vector comprising an Open Reading Frame (ORF) comprising sequences encoding for:
a) a *Chlamydomonas* carbonic anhydrase 1 (CAH1) secretion signal;
b) a target polypeptide; and
c) a luciferase.

2. The expression vector according to claim 1, wherein the CAH1 secretion signal has a sequence homology of at least 60 % to SEQ ID NO: 1.

3. The expression vector according to claim 1 or 2, wherein the target polypeptide is an anti-freeze peptide, a food ingredient, a bioremediation or industrial agent, a pharmaceutical ingredient, a therapeutic agent, a diagnostic agent or a vaccine.

4. The expression vector according to any one of claims 1 to 3, wherein the luciferase has a sequence homology of at least 60 % to SEQ ID NO: 3.

5. The expression vector according to any one of claims 1 to 4, wherein the ORF further comprises a sequence encoding for a protease cleavage site, in particular wherein said sequence encoding for a protease cleavage site is located between the sequence encoding for the target polypeptide and the sequence encoding for the luciferase.

6. The expression vector according to any one of claims 1 to 5, wherein the sequential arrangement of the encoding sequences in the ORF between the promoter and the terminator sequence is
a) 5'- X₁ - *cah1* - X₂ *-luc* - X₃ - *tps* - X₄ -3';
b) 5'- X₁ *- cah1 -* X₁- *tps -* X₃ - *luc* - X₄ -3',
wherein:
*cah1* is the sequence encoding for the CAH1 secretion signal,
*luc* is the sequence encoding for the luciferase,
*tps* is the sequence encoding for the target polypeptide, and
either X₁, X₂, X₃ and X₄ can independently from another encode for a spacer sequence, a protease cleavage site, an intron and/or a purification tag or may be missing,
in particular wherein
X₁ is an intron,
X₂ encodes for a spacer sequence or is missing,
X₃ encodes for a protease cleavage, optionally further comprising a spacer sequence, and
X₄ encodes for a spacer sequence and/or a purification tag such as a polyhistidine (polyHIS) tag or is missing.

7. The expression vector according to any one of claims 1 to 6, further comprising a heat shock protein promoter, preferably a heat shock protein 70A (HSP70A) promoter coupled to a ribulose-1,5-bisphosphate carboxylase/oxygenase small subunit (RBCS2) promoter.

8. The expression vector according to any one of claims 1 to 7, further comprising an enhancer, preferably wherein said enhancer is a ribulose-1/5-bisphosphatase (RBCS2) intronic enhancer, in particular wherein said expression vector comprises the combination of an HSP70A - RBCS2 promoter and an RBCS2 intronic enhancer.

9. The expression vector according to any one of claims 1 to 8, comprising a nucleic acid sequence having a sequence of at least 60 % sequence homology to SEQ ID NO: 5, in particular comprising a nucleic acid sequence encoding for a polypeptide having an amino acid sequence of at least 60 % sequence homology to SEQ ID NO: 6 or SEQ ID NO: 7.

10. A host cell comprising the expression vector according to any one of claims 1 to 9.

11. The host cell according to claim 10, wherein said host cell is a eukaryotic cell, preferably an autotrophic eukaryotic cell, more preferably a green alga cell, even more preferably a green alga cell, in particular a green alga cell **characterized in that** it lacks a cell wall and/or it is a green alga cell from the division of *Chlorophyta,* preferably a *Chlorophycea* cell, more preferably a *Volvocales* cell, even more preferably a *Chlamydomonadacea* cell, even more preferably a *Chlamydomona* cell, in particular a *Chlamydomonas reinhardtii* cell.

12. A method for producing a polypeptide, comprising the following steps:
i) providing a cell according to claim 10 or 11;
ii) cultivating said cell of step i) in a culture broth under conditions allowing the production and secretion of said polypeptide; and
iii) isolating said polypeptide from said culture broth or the direct use of that broth for the target recombinant polypeptide's application.

13. The method according to claim 12, wherein steps ii) and iii) are conducted simultaneously, in particular wherein cultivating is continuous cultivating.

14. The method according to claim 12 or 13, further comprising at least one of the steps selected from the group consisting of:
iv) detecting bioluminescence from the luciferase;
v) enzymatically cleaving the target polypeptide from the luciferase; and
vi) purifying said target polypeptide.

15. Use of a cell according to claim 10 or 11 for producing a polypeptide.


SEQUENCE LISTING

<110> Universität Bielefeld

<120> Expression Vector for a Secretion and Detection System

<130> P68227EP

<160> 21

<170> PatentIn version 3.5

<210> 1
<211> 21
<212> PRT
<213> Chlamydomonas reinhardtii

<400> 1

Met Ala Arg Thr Gly Ala Leu Leu Leu Val Ala Leu Ala Leu Ala Gly
1               5                   10                  15

Cys Ala Gln Ala Cys
            20

<210> 2
<211> 63
<212> DNA
<213> Chlamydomonas reinhardtii

<400> 2
atggcgcgta ctggcgctct actcctggtc gcgctggcgc ttgcgggctg cgcgcaggct 60

tgc 63

<210> 3
<211> 169
<212> PRT
<213> Gaussia princeps

<400> 3

Lys Pro Thr Glu Asn Asn Glu Asp Phe Asn Ile Val Ala Val Ala Ser
1               5                   10                  15

Asn Phe Ala Thr Thr Asp Leu Asp Ala Asp Arg Gly Lys Leu Pro Gly
            20                  25                  30

Lys Lys Leu Pro Leu Glu Val Leu Lys Glu Met Glu Ala Asn Ala Arg
        35                  40                  45

Lys Ala Gly Cys Thr Arg Gly Cys Leu Ile Cys Leu Ser His Ile Lys
    50                  55                  60

Cys Thr Pro Lys Met Lys Lys Phe Ile Pro Gly Arg Cys His Thr Tyr
65                  70                  75                  80

Glu Gly Asp Lys Glu Ser Ala Gln Gly Gly Ile Gly Glu Ala Ile Val
                85                  90                  95

Asp Ile Pro Glu Ile Pro Gly Phe Lys Asp Leu Glu Pro Met Glu Gln
            100                 105                 110

Phe Ile Ala Gln Val Asp Leu Cys Val Asp Cys Thr Thr Gly Cys Leu
        115                 120                 125

Lys Gly Leu Ala Asn Val Gln Cys Ser Asp Leu Leu Lys Lys Trp Leu
    130                 135                 140

Pro Gln Arg Cys Ala Thr Phe Ala Ser Lys Ile Gln Gly Gln Val Asp
145                 150                 155                 160

Lys Ile Lys Gly Ala Gly Gly Asp Thr
                165

<210> 4
<211> 507
<212> DNA
<213> Gaussia princeps

<400> 4
aagccgacgg agaacaacga ggacttcaac attgtcgccg tggcgtccaa ctttgccacc 60

accgatctgg acgccgaccg cggcaagctg ccgggcaaga agctgcccct ggaggtcctc 120

aaggaaatgg aggcgaacgc ccggaaggcc ggctgcaccc gcggctgcct gatctgcctg 180

tcccacatca agtgcacgcc gaagatgaag aagttcattc ccggccggtg ccacacctac 240

gagggcgaca aggagtccgc gcagggcggc attggcgagg cgatcgtgga catccccgag 300

attccgggct tcaaggacct ggagccgatg gagcagttca tcgcccaggt ggacctgtgc 360

gtggactgca cgaccggctg tctgaagggc ctggccaatg tgcagtgttc cgacctgctc 420

aagaagtggc tgccccagcg ctgtgccacc ttcgccagca agatccaggg ccaggtggac 480

aagattaagg gcgctggcgg cgatacg 507

<210> 5
<211> 576
<212> DNA
<213> artificial sequence

<220>
<223> encoding sequence for a fusion polypeptide of a CAH1 secretion
signal with gLuc (CAgLuc)

<400> 5
atggcgcgta ctggcgctct actcctggtc gcgctggcgc ttgcgggctg cgcgcaggct 60

tgcagatcta agccgacgga gaacaacgag gacttcaaca ttgtcgccgt ggcgtccaac 120

tttgccacca ccgatctgga cgccgaccgc ggcaagctgc cgggcaagaa gctgcccctg 180

gaggtcctca aggaaatgga ggcgaacgcc cggaaggccg gctgcacccg cggctgcctg 240

atctgcctgt cccacatcaa gtgcacgccg aagatgaaga agttcattcc cggccggtgc 300

cacacctacg agggcgacaa ggagtccgcg cagggcggca ttggcgaggc gatcgtggac 360

atccccgaga ttccgggctt caaggacctg gagccgatgg agcagttcat cgcccaggtg 420

gacctgtgcg tggactgcac gaccggctgt ctgaagggcc tggccaatgt gcagtgttcc 480

gacctgctca agaagtggct gccccagcgc tgtgccacct tcgccagcaa gatccagggc 540

caggtggaca agattaaggg cgctggcggc gatacg 576

<210> 6
<211> 324
<212> PRT
<213> artificial sequence

<220>
<223> fusion polypeptide with C-terminal HVRP and a polyHis tag
(CA-gLuc-HVRP)

<400> 6

Met Ala Arg Thr Gly Ala Leu Leu Leu Val Ala Leu Ala Leu Ala Gly
1               5                   10                  15

Cys Ala Gln Ala Cys Arg Ser Lys Pro Thr Glu Asn Asn Glu Asp Phe
            20                  25                  30

Asn Ile Val Ala Val Ala Ser Asn Phe Ala Thr Thr Asp Leu Asp Ala
        35                  40                  45

Asp Arg Gly Lys Leu Pro Gly Lys Lys Leu Pro Leu Glu Val Leu Lys
    50                  55                  60

Glu Met Glu Ala Asn Ala Arg Lys Ala Gly Cys Thr Arg Gly Cys Leu
65                  70                  75                  80

Ile Cys Leu Ser His Ile Lys Cys Thr Pro Lys Met Lys Lys Phe Ile
                85                  90                  95

Pro Gly Arg Cys His Thr Tyr Glu Gly Asp Lys Glu Ser Ala Gln Gly
            100                 105                 110

Gly Ile Gly Glu Ala Ile Val Asp Ile Pro Glu Ile Pro Gly Phe Lys
        115                 120                 125

Asp Leu Glu Pro Met Glu Gln Phe Ile Ala Gln Val Asp Leu Cys Val
    130                 135                 140

Asp Cys Thr Thr Gly Cys Leu Lys Gly Leu Ala Asn Val Gln Cys Ser
145                 150                 155                 160

Asp Leu Leu Lys Lys Trp Leu Pro Gln Arg Cys Ala Thr Phe Ala Ser
                165                 170                 175

Lys Ile Gln Gly Gln Val Asp Lys Ile Lys Gly Ala Gly Gly Asp Thr
            180                 185                 190

Ile Glu Gly Arg Asp Ile Asp Glu Gln Pro Asn Thr Ile Ser Gly Ser
        195                 200                 205

Asn Asn Thr Val Arg Ser Gly Ser Lys Asn Val Leu Ala Gly Asn Asp
    210                 215                 220

Asn Thr Val Ile Ser Gly Asp Asn Asn Ser Val Ser Gly Ser Asn Asn
225                 230                 235                 240

Thr Val Val Ser Gly Asn Asp Asn Thr Val Thr Gly Ser Asn His Val
                245                 250                 255

Val Ser Gly Thr Asn His Ile Val Thr Asp Asn Asn Asn Asn Val Ser
            260                 265                 270

Gly Asn Asp Asn Asn Val Ser Gly Ser Phe His Thr Val Ser Gly Gly
        275                 280                 285

His Asn Thr Val Ser Gly Ser Asn Asn Thr Val Ser Gly Ser Asn His
    290                 295                 300

Val Val Ser Gly Ser Asn Lys Val Val Thr Asp Ala Glu Phe His His
305                 310                 315                 320

His His His His

<210>  7
<211>  324
<212>  PRT
<213>  artificial sequence

<220>
<223>  fusion polypeptide with HVRP located between the CAH1 secretion
       signal an C-terminal gLuc and a polyHis tag (CA-HVRP-gLuc)

<400>  7

Met Ala Arg Thr Gly Ala Leu Leu Leu Val Ala Leu Ala Leu Ala Gly
1               5                   10                  15

Cys Ala Gln Ala Cys Arg Ser Asp Glu Gln Pro Asn Thr Ile Ser Gly
            20                  25                  30

Ser Asn Asn Thr Val Arg Ser Gly Ser Lys Asn Val Leu Ala Gly Asn
        35                  40                  45

Asp Asn Thr Val Ile Ser Gly Asp Asn Asn Ser Val Ser Gly Ser Asn
    50                  55                  60

Asn Thr Val Val Ser Gly Asn Asp Asn Thr Val Thr Gly Ser Asn His
65                  70                  75                  80

Val Val Ser Gly Thr Asn His Ile Val Thr Asp Asn Asn Asn Asn Val

                85                  90                  95

Ser Gly Asn Asp Asn Asn Val Ser Gly Ser Phe His Thr Val Ser Gly
            100                 105                 110

Gly His Asn Thr Val Ser Gly Ser Asn Asn Thr Val Ser Gly Ser Asn
        115                 120                 125

His Val Val Ser Gly Ser Asn Lys Val Val Thr Asp Ala Asp Ile Ile
    130                 135                 140

Glu Gly Arg Lys Pro Thr Glu Asn Asn Glu Asp Phe Asn Ile Val Ala
145                 150                 155                 160

Val Ala Ser Asn Phe Ala Thr Thr Asp Leu Asp Ala Asp Arg Gly Lys
                165                 170                 175

Leu Pro Gly Lys Lys Leu Pro Leu Glu Val Leu Lys Glu Met Glu Ala
            180                 185                 190

Asn Ala Arg Lys Ala Gly Cys Thr Arg Gly Cys Leu Ile Cys Leu Ser
        195                 200                 205

His Ile Lys Cys Thr Pro Lys Met Lys Lys Phe Ile Pro Gly Arg Cys
    210                 215                 220

His Thr Tyr Glu Gly Asp Lys Glu Ser Ala Gln Gly Gly Ile Gly Glu
225                 230                 235                 240

Ala Ile Val Asp Ile Pro Glu Ile Pro Gly Phe Lys Asp Leu Glu Pro
                245                 250                 255

Met Glu Gln Phe Ile Ala Gln Val Asp Leu Cys Val Asp Cys Thr Thr
            260                 265                 270

Gly Cys Leu Lys Gly Leu Ala Asn Val Gln Cys Ser Asp Leu Leu Lys
        275                 280                 285

Lys Trp Leu Pro Gln Arg Cys Ala Thr Phe Ala Ser Lys Ile Gln Gly
    290                 295                 300

Gln Val Asp Lys Ile Lys Gly Ala Gly Gly Asp Thr Glu Phe His His
305                 310                 315                 320

His His His His

<210> 8
<211> 27
<212> DNA
<213> artificial sequence

<220>
<223>  CAH1 primer FWNdeI

<400>  8
aattcatatg gcgcgtactg gcgctct                                          27


<210>  9
<211>  24
<212>  DNA
<213>  artificial sequence

<220>
<223>  CAH1 primer RVBg/II

<400>  9
aattagatct agcctgcgcg cagc                                             24


<210>  10
<211>  30
<212>  DNA
<213>  artificial sequence

<220>
<223>  gLuc primer FWNdeI

<400>  10
aattcatatg aagccgacgg agaacaacga                                       30


<210>  11
<211>  27
<212>  DNA
<213>  artificial sequence

<220>
<223>  gLuc primer RVEcoRV

<400>  11
aattgatatc cgtatcgccg ccagcgc                                          27


<210>  12
<211>  20
<212>  DNA
<213>  artificial sequence

<220>
<223>  forward primer

<400>  12
aagccgacgg agaacaacga                                                  20


<210>  13
<211>  17
<212>  DNA
<213>  artificial sequence

<220>
<223>  reverse primer

<400>  13
ggcgtcggtc accacct                                                     17


<210>  14

<211>   6944
<212>   DNA
<213>   artificial sequence

<220>
<223>   vector encoding for a fusion polypeptide

<400>   14
atagaccgag atagggttga gtgttgttcc agtttggaac aagagtccac tattaaagaa      60

cgtggactcc aacgtcaaag ggcgaaaaac cgtctatcag ggcgatggcc cactacgtga     120

accatcaccc taatcaagtt ttttggggtc gaggtgccgt aaagcactaa atcggaaccc     180

taaagggagc ccccgattta gagcttgacg gggaaagccg gcgaacgtgg cgagaaagga     240

agggaagaaa gcgaaaggag cgggcgctag ggcgctggca agtgtagcgg tcacgctgcg     300

cgtaaccacc acacccgccg cgcttaatgc gccgctacag ggcgcgtccc attcgccatt     360

caggctgcgc aactgttggg aagggcgatc ggtgcgggcc tcttcgctat tacgccagct     420

ggcgaaaggg ggatgtgctg caaggcgatt aagttgggta acgccagggt tttcccagtc     480

acgacgttgt aaaacgacgg ccagtgagcg cgcgtaatac gactcactat agggcgaatt     540

ggagctccac cgcggtggcg gccgctctag aactagtgct gaggcttgac atgattggtg     600

cgtatgtttg tatgaagcta caggactgat ttggcgggct atgagggcgg gggaagctct     660

ggaagggccg cgatggggcg cgcggcgtcc agaaggcgcc atacggcccg ctggcggcac     720

ccatccggta taaaagcccg cgaccccgaa cggtgacctc cactttcagc gacaaacgag     780

cacttataca tacgcgacta ttctgccgct atacataacc actcagctag cttaagatcc     840

catcaccggt gcatgccggg cgcgccagaa ggagcgcagc caaaccagga tgatgtttga     900

tggggtattt gagcacttgc aacccttatc cggaagcccc ctggcccaca aaggctaggc     960

gccaatgcaa gcagttcgca tgcagcccct ggagcggtgc cctcctgata aaccggccag    1020

ggggcctatg ttctttactt ttttacaaga gaagtcactc aacatcttaa aatggccagg    1080

tgagtcgacg agcaagcccg gcggatcagg cagcgtgctt gcagatttga cttgcaacgc    1140

ccgcattgtg tcgacgaagg cttttggctc ctctgtcgct gtctcaagca gcatctaacc    1200

ctgcgtcgcc gtttccattt gcaggatgca tatgaagccg acggagaaca acgaggactt    1260

caacattgtc gccgtggcgt ccaactttgc caccaccgat ctggacgccg accgcggcaa    1320

gctgccgggc aagaagctgc ccctggaggt cctcaaggaa atggaggcga acgcccggaa    1380

ggccggctgc acccgcggct gcctgatctg cctgtcccac atcaagtgca cgccgaagat    1440

gaagaagttc attcccggcc ggtgccacac ctacgagggc gacaaggagt ccgcgcaggg    1500

cggcattggc gaggcgatcg tggacatccc cgagattccg ggcttcaagg acctggagcc    1560

gatggagcag ttcatcgccc aggtggacct gtgcgtggac tgcacgaccg gctgtctgaa    1620

gggcctggcc aatgtgcagt gttccgacct gctcaagaag tggctgcccc agcgctgtgc    1680

caccttcgcc agcaagatcc agggccaggt ggacaagatt aagggcgctg gcggcgatac    1740

ggatatcgac gagcagccca acaccatcag cggcagcaac aacaccgtgc gcagcggcag    1800

caagaacgtg ctggccggca acgacaacac cgtgatcagc ggcgacaaca acagcgtgag 1860
cggcagcaac aacaccgtgg tgagcggcaa cgacaacacc gtgaccggca gcaaccacgt 1920
ggtgagcggc accaaccaca tcgtgaccga caacaacaac aacgtgagcg gcaacgacaa 1980
caacgtgagc ggcagcttcc acaccgtgag cggcggccac aacaccgtga gcggcagcaa 2040
caacaccgtg agcggcagca accacgtggt gagcggcagc aacaaggtgg tgaccgacgc 2100
ccaccaccac caccaccact aagaattccc gctccgtgta aatggaggcg ctcgttgatc 2160
tgagccttgc cccctgacga acggcggtgg atggaagata ctgctctcaa gtgctgaagc 2220
ggtagcttag ctccccgttt cgtgctgatc agtctttttc aacacgtaaa aagcggagga 2280
gttttgcaat tttgttggtt gtaacgatcc tccgttgatt ttggcctctt tctccatggg 2340
cgggctgggc gtatttgaag cggacccggg tcggtgtggg gtcggcccac ggtcaattag 2400
ccacaggagg atcaggggga ggtaggcacg tcgacttggt ttgcgacccc gcagttttgg 2460
cggacgtgct gttgtagatg ttagcgtgtg cgtgagccag tggccaacgt gccacaccca 2520
ttgagaagac caaccaactt actggcaata tctgccaatg ccatactgca tgtaatggcc 2580
aggccatgtg agagtttgcc gtgcctgcgc gcgccccggg ggcgcagttt agctgaccag 2640
ccgtgggatg atgcacgcat ttgcaaggac agggtaatca cagcagcaac atggtgggct 2700
taggacagct gtgggtcagt ggacggacgg caggggaggg acggcgcagc tcgggagaca 2760
gggggagaca gcgtgactgt gcacatcaag cttatcgata ccgtcgaccc tcgagcgggg 2820
agctcgctga ggcttgacat gattggtgcg tatgtttgta tgaagctaca ggactgattt 2880
ggcgggctat gagggcgcgg gaagctctgg aagggccgcg atggggcgcg cggcgtccag 2940
aaggcgccat acggcccgct ggcggcaccc atccggtata aaagcccgcg accccgaacg 3000
gtgacctcca ctttcagcga caaacgagca cttatacata cgcgactatt ctgccgctat 3060
acataaccac tcagctagct taagatccca tcaagcttgc atgccgggcg cgccagaagg 3120
agcgcagcca aaccaggatg atgtttgatg gggtatttga gcacttgcaa cccttatccg 3180
gaagccccct ggcccacaaa ggctaggcgc caatgcaagc agttcgcatg cagcccctgg 3240
agcggtgccc tcctgataaa ccggccaggg ggcctatgtt ctttactttt ttacaagaga 3300
agtcactcaa catcttaaaa tggccaggtg agtcgacgag caagcccggc ggatcaggca 3360
gcgtgcttgc agatttgact tgcaacgccc gcattgtgtc gacgaaggct tttggctcct 3420
ctgtcgctgt ctcaagcagc atctaaccct gcgtcgccgt ttccatttgc aggatggcca 3480
ctccgccctc cccggtgctg aagaatttcg aagcatggac gatgcgttgc gtgcactgcg 3540
gggtcggtat cccggttgtg agtgggttgt tgtggaggat ggggcctcgg gggctggtgt 3600
ttatcggctt cggggtggtg ggcgggagtt gtttgtcaag gtggcagctc tgggggccgg 3660
ggtgggcttg ttgggtgagg ctgagcggct ggtgtggttg gcggaggtgg ggattcccgt 3720
acctcgtgtt gtggagggtg gtggggacga gagggtcgcc tggttggtca ccgaagcggt 3780
tccggggcgt ccggccagtg cgcggtggcc gcgggagcag cggctggacg tggcggtggc 3840

gctcgcgggg ctcgctcgtt cgctgcacgc gctggactgg gagcggtgtc cgttcgatcg 3900
cagtctcgcg gtgacggtgc cgcaggcggc ccgtgctgtc gctgaaggga gcgtcgactt 3960
ggaggatctg gacgaggagc ggaaggggtg gtcgggggag cggcttctcg ccgagctgga 4020
gcggactcgg cctgcggacg aggatctggc ggtttgccac ggtcacctgt gcccggacaa 4080
cgtgctgctc gaccctcgta cctgcgaggt gaccgggctg atcgacgtgg ggcgggtcgg 4140
ccgtgcggac cggcactccg atctcgcgct ggtgctgcgc gagctggccc acgaggagga 4200
cccgtggttc gggccggagt gttccgcggc gttcctgcgg gagtacgggc gcgggtggga 4260
tggggcggta tcggaggaaa agctggcgtt ttaccggctg ttggacgagt tcttctgagg 4320
gacctgatgg tgttggtggc tgggtagggt tgcgtcgcgt gggtgacagc acagtgtgga 4380
cgttgggatc cccgctccgt gtaaatggag gcgctcgttg atctgagcct tgccccctga 4440
cgaacggcgg tggatggaag atactgctct caagtgctga agcggtagct tagctccccg 4500
tttcgtgctg atcagtcttt ttcaacacgt aaaaagcgga ggagttttgc aattttgttg 4560
gttgtaacga tcctccgttg attttggcct ctttctccat gggcgggctg ggcgtatttg 4620
aagcgggtac ccagcttttg ttccctttag tgagggttaa ttgcgcgctt ggcgtaatca 4680
tggtcatagc tgtttcctgt gtgaaattgt tatccgctca caattccaca caacatacga 4740
gccggaagca taaagtgtaa agcctggggt gcctaatgag tgagctaact cacattaatt 4800
gcgttgcgct cactgcccgc tttccagtcg ggaaacctgt cgtgccagct gcattaatga 4860
atcggccaac gcgcggggag aggcggtttg cgtattgggc gctcttccgc ttcctcgctc 4920
actgactcgc tgcgctcggt cgttcggctg cggcgagcgg tatcagctca ctcaaaggcg 4980
gtaatacggt tatccacaga atcaggggat aacgcaggaa agaacatgtg agcaaaaggc 5040
cagcaaaagg ccaggaaccg taaaaaggcc gcgttgctgg cgtttttcca taggctccgc 5100
ccccctgacg agcatcacaa aaatcgacgc tcaagtcaga ggtggcgaaa cccgacagga 5160
ctataaagat accaggcgtt tccccctgga agctccctcg tgcgctctcc tgttccgacc 5220
ctgccgctta ccggatacct gtccgccttt ctcccttcgg gaagcgtggc gctttctcat 5280
agctcacgct gtaggtatct cagttcggtg taggtcgttc gctccaagct gggctgtgtg 5340
cacgaacccc ccgttcagcc cgaccgctgc gccttatccg gtaactatcg tcttgagtcc 5400
aacccggtaa gacacgactt atcgccactg gcagcagcca ctggtaacag gattagcaga 5460
gcgaggtatg taggcggtgc tacagagttc ttgaagtggt ggcctaacta cggctacact 5520
agaaggacag tatttggtat ctgcgctctg ctgaagccag ttaccttcgg aaaaagagtt 5580
ggtagctctt gatccggcaa acaaaccacc gctggtagcg gtggtttttt tgtttgcaag 5640
cagcagatta cgcgcagaaa aaaaggatct caagaagatc ctttgatctt ttctacgggg 5700
tctgacgctc agtggaacga aaactcacgt taagggattt tggtcatgag attatcaaaa 5760
aggatcttca cctagatcct tttaaattaa aaatgaagtt ttaaatcaat ctaaagtata 5820
tatgagtaaa cttggtctga cagttaccaa tgcttaatca gtgaggcacc tatctcagcg 5880

atctgtctat ttcgttcatc catagttgcc tgactccccg tcgtgtagat aactacgata 5940

cgggagggct taccatctgg ccccagtgct gcaatgatac cgcgagaccc acgctcaccg 6000

gctccagatt tatcagcaat aaaccagcca gccggaaggg ccgagcgcag aagtggtcct 6060

gcaactttat ccgcctccat ccagtctatt aattgttgcc gggaagctag agtaagtagt 6120

tcgccagtta atagtttgcg caacgttgtt gccattgcta caggcatcgt ggtgtcacgc 6180

tcgtcgtttg gtatggcttc attcagctcc ggttcccaac gatcaaggcg agttacatga 6240

tcccccatgt tgtgcaaaaa agcggttagc tccttcggtc ctccgatcgt tgtcagaagt 6300

aagttggccg cagtgttatc actcatggtt atggcagcac tgcataattc tcttactgtc 6360

atgccatccg taagatgctt ttctgtgact ggtgagtact caaccaagtc attctgagaa 6420

tagtgtatgc ggcgaccgag ttgctcttgc ccggcgtcaa tacgggataa taccgcgcca 6480

catagcagaa ctttaaaagt gctcatcatt ggaaaacgtt cttcggggcg aaaactctca 6540

aggatcttac cgctgttgag atccagttcg atgtaaccca ctcgtgcacc caactgatct 6600

tcagcatctt ttactttcac cagcgtttct gggtgagcaa aaacaggaag gcaaaatgcc 6660

gcaaaaaagg gaataagggc gacacggaaa tgttgaatac tcatactctt cctttttcaa 6720

tattattgaa gcatttatca gggttattgt ctcatgagcg gatacatatt tgaatgtatt 6780

tagaaaaata aacaaatagg ggttccgcgc acatttcccc gaaaagtgcc acactaaatt 6840

gtaagcgtta atattttgtt aaaattcgcg ttaaattttt gttaaatcag ctcatttttt 6900

aaccaatagg ccgaaatcgg caaaatccct tataaatcaa aaga 6944

<210> 15
<211> 7069
<212> DNA
<213> artificial sequence

<220>
<223> vector encoding for a fusion polypeptide

<400> 15
atagaccgag atagggttga gtgttgttcc agtttggaac aagagtccac tattaaagaa 60

cgtggactcc aacgtcaaag ggcgaaaaac cgtctatcag ggcgatggcc cactacgtga 120

accatcaccc taatcaagtt ttttggggtc gaggtgccgt aaagcactaa atcggaaccc 180

taaagggagc ccccgattta gagcttgacg gggaaagccg gcgaacgtgg cgagaaagga 240

agggaagaaa gcgaaaggag cgggcgctag ggcgctggca agtgtagcgg tcacgctgcg 300

cgtaaccacc acacccgccg cgcttaatgc gccgctacag ggcgcgtccc attcgccatt 360

caggctgcgc aactgttggg aagggcgatc ggtgcgggcc tcttcgctat tacgccagct 420

ggcgaaaggg ggatgtgctg caaggcgatt aagttgggta acgccagggt tttcccagtc 480

acgacgttgt aaaacgacgg ccagtgagcg cgcgtaatac gactcactat agggcgaatt 540

ggagctccac cgcggtggcg gccgctctag aactagtgct gaggcttgac atgattggtg 600

cgtatgtttg tatgaagcta caggactgat ttggcgggct atgagggcgg gggaagctct 660

ggaagggccg cgatggggcg cgcggcgtcc agaaggcgcc atacggcccg ctggcggcac 720
ccatccggta taaaagcccg cgaccccgaa cggtgacctc cactttcagc gacaaacgag 780
cacttataca tacgcgacta ttctgccgct atacataacc actcagctag cttaagatcc 840
catcaccggt gcatgccggg cgcgccagaa ggagcgcagc caaaccagga tgatgtttga 900
tggggtattt gagcacttgc aacccttatc cggaagcccc ctggcccaca aaggctaggc 960
gccaatgcaa gcagttcgca tgcagcccct ggagcggtgc cctcctgata aaccggccag 1020
ggggcctatg ttctttactt ttttacaaga gaagtcactc aacatcttaa aatggccagg 1080
tgagtcgacg agcaagcccg gcggatcagg cagcgtgctt gcagatttga cttgcaacgc 1140
ccgcattgtg tcgacgaagg cttttggctc ctctgtcgct gtctcaagca gcatctaacc 1200
ctgcgtcgcc gtttccattt gcaggatgca tatgggcgtg aaggtgctgt tcgccctgat 1260
ctgcatcgcc gtcgccgagg cgagatctaa gccgacggag aacaacgagg acttcaacat 1320
tgtcgccgtg gcgtccaact ttgccaccac cgatctggac gccgaccgcg gcaagctgcc 1380
gggcaagaag ctgcccctgg aggtcctcaa ggaaatggag gcgaacgccc ggaaggccgg 1440
ctgcacccgc ggctgcctga tctgcctgtc ccacatcaag tgcacgccga agatgaagaa 1500
gttcattccc ggccggtgcc acacctacga gggcgacaag gagtccgcgc agggcggcat 1560
tggcgaggcg atcgtggaca tccccgagat tccgggcttc aaggacctgg agccgatgga 1620
gcagttcatc gcccaggtgg acctgtgcgt ggactgcacg accggctgtc tgaagggcct 1680
ggccaatgtg cagtgttccg acctgctcaa gaagtggctg ccccagcgct gtgccacctt 1740
cgccagcaag atccagggcc aggtggacaa gattaagggc gctggcggcg atacggatat 1800
cgacgagcag cccaacacca tcagcggcag caacaacacc gtgcgcagcg gcagcaagaa 1860
cgtgctggcc ggcaacgaca acaccgtgat cagcggcgac aacaacagcg tgagcggcag 1920
caacaacacc gtggtgagcg gcaacgacaa caccgtgacc ggcagcaacc acgtggtgag 1980
cggcaccaac cacatcgtga ccgacaacaa caacaacgtg agcggcaacg acaacaacgt 2040
gagcggcagc ttccacaccg tgagcggcgg ccacaacacc gtgagcggca gcaacaacac 2100
cgtgagcggc agcaaccacg tggtgagcgg cagcaacaag gtggtgaccg acgcccacca 2160
ccaccaccac cactaagaat tcccgctccg tgtaaatgga ggcgctcgtt gatctgagcc 2220
ttgccccctg acgaacggcg gtggatggaa gatactgctc tcaagtgctg aagcggtagc 2280
ttagctcccc gtttcgtgct gatcagtctt tttcaacacg taaaaagcgg aggagttttg 2340
caattttgtt ggttgtaacg atcctccgtt gattttggcc tctttctcca tgggcgggct 2400
gggcgtattt gaagcggacc cgggtcggtg tggggtcggc ccacggtcaa ttagccacag 2460
gaggatcagg gggaggtagg cacgtcgact tggtttgcga ccccgcagtt ttggcggacg 2520
tgctgttgta gatgttagcg tgtgcgtgag ccagtggcca acgtgccaca cccattgaga 2580
agaccaacca acttactggc aatatctgcc aatgccatac tgcatgtaat ggccaggcca 2640
tgtgagagtt tgccgtgcct gcgcgcgccc cgggggcggg ggggggacgg gtggggggta 2700

gggggtctca cgggaacagc acgctagggg tcaggggggg gggggggcgc agtttagctg 2760
accagccgtg ggatgatgca cgcatttgca aggacagggt aatcacagca gcaacatggt 2820
gggcttagga cagctgtggg tcagtggacg gacggcaggg gagggacggc gcagctcggg 2880
agacaggggg agacagcgtg actgtgcaca tcaagcttat cgataccgtc gaccctcgag 2940
cggggagctc gctgaggctt gacatgattg gtgcgtatgt ttgtatgaag ctacaggact 3000
gatttggcgg gctatgaggg cgcgggaagc tctggaaggg ccgcgatggg gcgcgcggcg 3060
tccagaaggc gccatacggc ccgctggcgg cacccatccg gtataaaagc ccgcgacccc 3120
gaacggtgac ctccactttc agcgacaaac gagcacttat acatacgcga ctattctgcc 3180
gctatacata accactcagc tagcttaaga tcccatcaag cttgcatgcc gggcgcgcca 3240
gaaggagcgc agccaaacca ggatgatgtt tgatggggta tttgagcact tgcaaccctt 3300
atccggaagc cccctggccc acaaaggcta ggcgccaatg caagcagttc gcatgcagcc 3360
cctggagcgg tgccctcctg ataaaccggc cagggggcct atgttcttta cttttttaca 3420
agagaagtca ctcaacatct taaaatggcc aggtgagtcg acgagcaagc ccggcggatc 3480
aggcagcgtg cttgcagatt tgacttgcaa cgcccgcatt gtgtcgacga aggcttttgg 3540
ctcctctgtc gctgtctcaa gcagcatcta accctgcgtc gccgtttcca tttgcaggat 3600
ggccactccg ccctccccgg tgctgaagaa tttcgaagca tggacgatgc gttgcgtgca 3660
ctgcggggtc ggtatcccgg ttgtgagtgg gttgttgtgg aggatggggc ctcgggggct 3720
ggtgtttatc ggcttcgggg tggtgggcgg gagttgtttg tcaaggtggc agctctgggg 3780
gccggggtgg gcttgttggg tgaggctgag cggctggtgt ggttggcgga ggtggggatt 3840
cccgtacctc gtgttgtgga gggtggtggg gacgagaggg tcgcctggtt ggtcaccgaa 3900
gcggttccgg ggcgtccggc cagtgcgcgg tggccgcggg agcagcggct ggacgtggcg 3960
gtggcgctcg cggggctcgc tcgttcgctg cacgcgctgg actgggagcg gtgtccgttc 4020
gatcgcagtc tcgcggtgac ggtgccgcag gcggcccgtg ctgtcgctga agggagcgtc 4080
gacttggagg atctggacga ggagcggaag gggtggtcgg gggagcggct tctcgccgag 4140
ctggagcgga ctcggcctgc ggacgaggat ctggcggttt gccacggtca cctgtgcccg 4200
gacaacgtgc tgctcgaccc tcgtacctgc gaggtgaccg ggctgatcga cgtggggcgg 4260
gtcggccgtg cggaccggca ctccgatctc gcgctggtgc tgcgcgagct ggcccacgag 4320
gaggacccgt ggttcgggcc ggagtgttcc gcggcgttcc tgcgggagta cgggcgcggg 4380
tgggatgggg cggtatcgga ggaaaagctg gcgttttacc ggctgttgga cgagttcttc 4440
tgagggacct gatggtgttg gtggctgggt agggttgcgt cgcgtgggtg acagcacagt 4500
gtggacgttg ggatccccgc tccgtgtaaa tggaggcgct cgttgatctg agccttgccc 4560
cctgacgaac ggcggtggat ggaagatact gctctcaagt gctgaagcgg tagcttagct 4620
ccccgtttcg tgctgatcag tctttttcaa cacgtaaaaa gcggaggagt tttgcaattt 4680
tgttggttgt aacgatcctc cgttgatttt ggcctctttc tccatgggcg ggctgggcgt 4740

atttgaagcg ggtacccagc ttttgttccc tttagtgagg gttaattgcg cgcttggcgt 4800
aatcatggtc atagctgttt cctgtgtgaa attgttatcc gctcacaatt ccacacaaca 4860
tacgagccgg aagcataaag tgtaaagcct ggggtgccta atgagtgagc taactcacat 4920
taattgcgtt gcgctcactg cccgctttcc agtcgggaaa cctgtcgtgc cagctgcatt 4980
aatgaatcgg ccaacgcgcg gggagaggcg gtttgcgtat tgggcgctct tccgcttcct 5040
cgctcactga ctcgctgcgc tcggtcgttc ggctgcggcg agcggtatca gctcactcaa 5100
aggcggtaat acggttatcc acagaatcag gggataacgc aggaaagaac atgtgagcaa 5160
aaggccagca aaaggccagg aaccgtaaaa aggccgcgtt gctggcgttt ttccataggc 5220
tccgcccccc tgacgagcat cacaaaaatc gacgctcaag tcagaggtgg cgaaacccga 5280
caggactata aagataccag gcgtttcccc ctggaagctc cctcgtgcgc tctcctgttc 5340
cgaccctgcc gcttaccgga tacctgtccg cctttctccc ttcgggaagc gtggcgcttt 5400
ctcatagctc acgctgtagg tatctcagtt cggtgtaggt cgttcgctcc aagctgggct 5460
gtgtgcacga accccccgtt cagcccgacc gctgcgcctt atccggtaac tatcgtcttg 5520
agtccaaccc ggtaagacac gacttatcgc cactggcagc agccactggt aacaggatta 5580
gcagagcgag gtatgtaggc ggtgctacag agttcttgaa gtggtggcct aactacggct 5640
acactagaag gacagtattt ggtatctgcg ctctgctgaa gccagttacc ttcggaaaaa 5700
gagttggtag ctcttgatcc ggcaaacaaa ccaccgctgg tagcggtggt ttttttgttt 5760
gcaagcagca gattacgcgc agaaaaaaag gatctcaaga agatcctttg atcttttcta 5820
cggggtctga cgctcagtgg aacgaaaact cacgttaagg gattttggtc atgagattat 5880
caaaaaggat cttcacctag atccttttaa attaaaaatg aagttttaaa tcaatctaaa 5940
gtatatatga gtaaacttgg tctgacagtt accaatgctt aatcagtgag gcacctatct 6000
cagcgatctg tctatttcgt tcatccatag ttgcctgact ccccgtcgtg tagataacta 6060
cgatacggga gggcttacca tctggcccca gtgctgcaat gataccgcga gacccacgct 6120
caccggctcc agatttatca gcaataaacc agccagccgg aagggccgag cgcagaagtg 6180
gtcctgcaac tttatccgcc tccatccagt ctattaattg ttgccgggaa gctagagtaa 6240
gtagttcgcc agttaatagt ttgcgcaacg ttgttgccat tgctacaggc atcgtggtgt 6300
cacgctcgtc gtttggtatg gcttcattca gctccggttc ccaacgatca aggcgagtta 6360
catgatcccc catgttgtgc aaaaaagcgg ttagctcctt cggtcctccg atcgttgtca 6420
gaagtaagtt ggccgcagtg ttatcactca tggttatggc agcactgcat aattctctta 6480
ctgtcatgcc atccgtaaga tgcttttctg tgactggtga gtactcaacc aagtcattct 6540
gagaatagtg tatgcggcga ccgagttgct cttgcccggc gtcaatacgg gataataccg 6600
cgccacatag cagaacttta aaagtgctca tcattggaaa acgttcttcg gggcgaaaac 6660
tctcaaggat cttaccgctg ttgagatcca gttcgatgta acccactcgt gcacccaact 6720
gatcttcagc atcttttact ttcaccagcg tttctgggtg agcaaaaaca ggaaggcaaa 6780

atgccgcaaa aaagggaata agggcgacac ggaaatgttg aatactcata ctcttccttt 6840

ttcaatatta ttgaagcatt tatcagggtt attgtctcat gagcggatac atatttgaat 6900

gtatttagaa aaataaacaa ataggggttc cgcgcacatt tccccgaaaa gtgccacact 6960

aaattgtaag cgttaatatt ttgttaaaat tcgcgttaaa tttttgttaa atcagctcat 7020

tttttaacca ataggccgaa atcggcaaaa tcccttataa atcaaaaga 7069

<210> 16
<211> 7010
<212> DNA
<213> artificial sequence

<220>
<223> vector encoding for a fusion peptide

<400> 16
atagaccgag atagggttga gtgttgttcc agtttggaac aagagtccac tattaaagaa 60

cgtggactcc aacgtcaaag ggcgaaaaac cgtctatcag ggcgatggcc cactacgtga 120

accatcaccc taatcaagtt ttttggggtc gaggtgccgt aaagcactaa atcggaaccc 180

taaagggagc ccccgattta gagcttgacg gggaaagccg gcgaacgtgg cgagaaagga 240

agggaagaaa gcgaaaggag cgggcgctag ggcgctggca agtgtagcgg tcacgctgcg 300

cgtaaccacc acacccgccg cgcttaatgc gccgctacag ggcgcgtccc attcgccatt 360

caggctgcgc aactgttggg aagggcgatc ggtgcgggcc tcttcgctat tacgccagct 420

ggcgaaaggg ggatgtgctg caaggcgatt aagttgggta acgccagggt tttcccagtc 480

acgacgttgt aaaacgacgg ccagtgagcg cgcgtaatac gactcactat agggcgaatt 540

ggagctccac cgcggtggcg gccgctctag aactagtgct gaggcttgac atgattggtg 600

cgtatgtttg tatgaagcta caggactgat ttggcgggct atgagggcgg gggaagctct 660

ggaagggccg cgatggggcg cgcggcgtcc agaaggcgcc atacggcccg ctggcggcac 720

ccatccggta taaaagcccg cgaccccgaa cggtgacctc cactttcagc gacaaacgag 780

cacttataca tacgcgacta ttctgccgct atacataacc actcagctag cttaagatcc 840

catcaccggt gcatgccggg cgcgccagaa ggagcgcagc caaaccagga tgatgtttga 900

tggggtattt gagcacttgc aacccttatc cggaagcccc ctggcccaca aaggctaggc 960

gccaatgcaa gcagttcgca tgcagcccct ggagcggtgc cctcctgata aaccggccag 1020

ggggcctatg ttctttactt ttttacaaga gaagtcactc aacatcttaa aatggccagg 1080

tgagtcgacg agcaagcccg gcggatcagg cagcgtgctt gcagatttga cttgcaacgc 1140

ccgcattgtg tcgacgaagg cttttggctc ctctgtcgct gtctcaagca gcatctaacc 1200

ctgcgtcgcc gtttccattt gcaggatgca tatggcgcgt actggcgctc tactcctggt 1260

cgcgctggcg cttgcgggct gcgcgcaggc ttgcagatct aagccgacgg agaacaacga 1320

ggacttcaac attgtcgccg tggcgtccaa ctttgccacc accgatctgg acgccgaccg 1380

cggcaagctg ccgggcaaga agctgcccct ggaggtcctc aaggaaatgg aggcgaacgc 1440

ccggaaggcc ggctgcaccc gcggctgcct gatctgcctg tcccacatca agtgcacgcc 1500
gaagatgaag aagttcattc ccggccggtg ccacacctac gagggcgaca aggagtccgc 1560
gcagggcggc attggcgagg cgatcgtgga catccccgag attccgggct tcaaggacct 1620
ggagccgatg gagcagttca tcgcccaggt ggacctgtgc gtggactgca cgaccggctg 1680
tctgaagggc ctggccaatg tgcagtgttc cgacctgctc aagaagtggc tgccccagcg 1740
ctgtgccacc ttcgccagca agatccaggg ccaggtggac aagattaagg gcgctggcgg 1800
cgatacggat atcgacgagc agcccaacac catcagcggc agcaacaaca ccgtgcgcag 1860
cggcagcaag aacgtgctgg ccggcaacga caacaccgtg atcagcggcg acaacaacag 1920
cgtgagcggc agcaacaaca ccgtggtgag cggcaacgac aacaccgtga ccggcagcaa 1980
ccacgtggtg agcggcacca accacatcgt gaccgacaac aacaacaacg tgagcggcaa 2040
cgacaacaac gtgagcggca gcttccacac cgtgagcggc ggccacaaca ccgtgagcgg 2100
cagcaacaac accgtgagcg gcagcaacca cgtggtgagc ggcagcaaca aggtggtgac 2160
cgacgcccac caccaccacc accactaaga attcccgctc cgtgtaaatg gaggcgctcg 2220
ttgatctgag ccttgccccc tgacgaacgg cggtggatgg aagatactgc tctcaagtgc 2280
tgaagcggta gcttagctcc ccgtttcgtg ctgatcagtc tttttcaaca cgtaaaaagc 2340
ggaggagttt tgcaattttg ttggttgtaa cgatcctccg ttgattttgg cctctttctc 2400
catgggcggg ctgggcgtat ttgaagcgga cccgggtcgg tgtggggtcg gcccacggtc 2460
aattagccac aggaggatca gggggaggta ggcacgtcga cttggtttgc gaccccgcag 2520
ttttggcgga cgtgctgttg tagatgttag cgtgtgcgtg agccagtggc caacgtgcca 2580
cacccattga gaagaccaac caacttactg gcaatatctg ccaatgccat actgcatgta 2640
atggccaggc catgtgagag tttgccgtgc ctgcgcgcgc cccgggggcg cagtttagct 2700
gaccagccgt gggatgatgc acgcatttgc aaggacaggg taatcacagc agcaacatgg 2760
tgggcttagg acagctgtgg gtcagtggac ggacggcagg ggagggacgg cgcagctcgg 2820
gagacagggg gagacagcgt gactgtgcac atcaagctta tcgataccgt cgaccctcga 2880
gcggggagct cgctgaggct tgacatgatt ggtgcgtatg tttgtatgaa gctacaggac 2940
tgatttggcg ggctatgagg gcgcgggaag ctctggaagg gccgcgatgg ggcgcgcggc 3000
gtccagaagg cgccatacgg cccgctggcg gcacccatcc ggtataaaag cccgcgaccc 3060
cgaacggtga cctccacttt cagcgacaaa cgagcactta tacatacgcg actattctgc 3120
cgctatacat aaccactcag ctagcttaag atcccatcaa gcttgcatgc cgggcgcgcc 3180
agaaggagcg cagccaaacc aggatgatgt ttgatggggt atttgagcac ttgcaaccct 3240
tatccggaag ccccctggcc cacaaaggct aggcgccaat gcaagcagtt cgcatgcagc 3300
ccctggagcg gtgccctcct gataaaccgg ccagggggcc tatgttcttt acttttttac 3360
aagagaagtc actcaacatc ttaaaatggc caggtgagtc gacgagcaag cccggcggat 3420
caggcagcgt gcttgcagat ttgacttgca acgcccgcat tgtgtcgacg aaggcttttg 3480

gctcctctgt cgctgtctca agcagcatct aaccctgcgt cgccgtttcc atttgcagga 3540
tggccactcc gccctccccg gtgctgaaga atttcgaagc atggacgatg cgttgcgtgc 3600
actgcggggt cggtatcccg gttgtgagtg ggttgttgtg gaggatgggg cctcgggggc 3660
tggtgtttat cggcttcggg gtggtgggcg ggagttgttt gtcaaggtgg cagctctggg 3720
ggccggggtg ggcttgttgg gtgaggctga gcggctggtg tggttggcgg aggtggggat 3780
tcccgtacct cgtgttgtgg agggtggtgg ggacgagagg gtcgcctggt tggtcaccga 3840
agcggttccg gggcgtccgg ccagtgcgcg gtggccgcgg gagcagcggc tggacgtggc 3900
ggtggcgctc gcggggctcg ctcgttcgct gcacgcgctg gactgggagc ggtgtccgtt 3960
cgatcgcagt ctcgcggtga cggtgccgca ggcggcccgt gctgtcgctg aagggagcgt 4020
cgacttggag gatctggacg aggagcggaa ggggtggtcg ggggagcggc ttctcgccga 4080
gctggagcgg actcggcctg cggacgagga tctggcggtt tgccacggtc acctgtgccc 4140
ggacaacgtg ctgctcgacc ctcgtacctg cgaggtgacc gggctgatcg acgtggggcg 4200
ggtcggccgt gcggaccggc actccgatct cgcgctggtg ctgcgcgagc tggcccacga 4260
ggaggacccg tggttcgggc cggagtgttc cgcggcgttc ctgcgggagt acgggcgcgg 4320
gtgggatggg gcggtatcgg aggaaaagct ggcgttttac cggctgttgg acgagttctt 4380
ctgagggacc tgatggtgtt ggtggctggg tagggttgcg tcgcgtgggt gacagcacag 4440
tgtggacgtt gggatccccg ctccgtgtaa atggaggcgc tcgttgatct gagccttgcc 4500
ccctgacgaa cggcggtgga tggaagatac tgctctcaag tgctgaagcg gtagcttagc 4560
tccccgtttc gtgctgatca gtctttttca acacgtaaaa agcggaggag ttttgcaatt 4620
ttgttggttg taacgatcct ccgttgattt tggcctcttt ctccatgggc gggctgggcg 4680
tatttgaagc gggtacccag cttttgttcc ctttagtgag ggttaattgc gcgcttggcg 4740
taatcatggt catagctgtt tcctgtgtga aattgttatc cgctcacaat tccacacaac 4800
atacgagccg gaagcataaa gtgtaaagcc tggggtgcct aatgagtgag ctaactcaca 4860
ttaattgcgt tgcgctcact gcccgctttc cagtcgggaa acctgtcgtg ccagctgcat 4920
taatgaatcg gccaacgcgc ggggagaggc ggtttgcgta ttgggcgctc ttccgcttcc 4980
tcgctcactg actcgctgcg ctcggtcgtt cggctgcggc gagcggtatc agctcactca 5040
aaggcggtaa tacggttatc cacagaatca ggggataacg caggaaagaa catgtgagca 5100
aaaggccagc aaaaggccag gaaccgtaaa aaggccgcgt tgctggcgtt tttccatagg 5160
ctccgccccc ctgacgagca tcacaaaaat cgacgctcaa gtcagaggtg gcgaaacccg 5220
acaggactat aaagatacca ggcgtttccc cctggaagct ccctcgtgcg ctctcctgtt 5280
ccgaccctgc cgcttaccgg atacctgtcc gcctttctcc cttcgggaag cgtggcgctt 5340
tctcatagct cacgctgtag gtatctcagt tcggtgtagg tcgttcgctc caagctgggc 5400
tgtgtgcacg aaccccccgt tcagcccgac cgctgcgcct tatccggtaa ctatcgtctt 5460
gagtccaacc cggtaagaca cgacttatcg ccactggcag cagccactgg taacaggatt 5520

agcagagcga ggtatgtagg cggtgctaca gagttcttga agtggtggcc taactacggc 5580

tacactagaa ggacagtatt tggtatctgc gctctgctga agccagttac cttcggaaaa 5640

agagttggta gctcttgatc cggcaaacaa accaccgctg gtagcggtgg tttttttgtt 5700

tgcaagcagc agattacgcg cagaaaaaaa ggatctcaag aagatccttt gatcttttct 5760

acggggtctg acgctcagtg gaacgaaaac tcacgttaag ggattttggt catgagatta 5820

tcaaaaagga tcttcaccta gatcctttta aattaaaaat gaagttttaa atcaatctaa 5880

agtatatatg agtaaacttg gtctgacagt taccaatgct taatcagtga ggcacctatc 5940

tcagcgatct gtctatttcg ttcatccata gttgcctgac tccccgtcgt gtagataact 6000

acgatacggg agggcttacc atctggcccc agtgctgcaa tgataccgcg agacccacgc 6060

tcaccggctc cagatttatc agcaataaac cagccagccg gaagggccga gcgcagaagt 6120

ggtcctgcaa ctttatccgc ctccatccag tctattaatt gttgccggga agctagagta 6180

agtagttcgc cagttaatag tttgcgcaac gttgttgcca ttgctacagg catcgtggtg 6240

tcacgctcgt cgtttggtat ggcttcattc agctccggtt cccaacgatc aaggcgagtt 6300

acatgatccc ccatgttgtg caaaaaagcg gttagctcct tcggtcctcc gatcgttgtc 6360

agaagtaagt tggccgcagt gttatcactc atggttatgg cagcactgca taattctctt 6420

actgtcatgc catccgtaag atgcttttct gtgactggtg agtactcaac caagtcattc 6480

tgagaatagt gtatgcggcg accgagttgc tcttgcccgg cgtcaatacg ggataatacc 6540

gcgccacata gcagaacttt aaaagtgctc atcattggaa aacgttcttc ggggcgaaaa 6600

ctctcaagga tcttaccgct gttgagatcc agttcgatgt aacccactcg tgcacccaac 6660

tgatcttcag catcttttac tttcaccagc gtttctgggt gagcaaaaac aggaaggcaa 6720

aatgccgcaa aaaagggaat aagggcgaca cggaaatgtt gaatactcat actcttcctt 6780

tttcaatatt attgaagcat ttatcagggt tattgtctca tgagcggata catatttgaa 6840

tgtatttaga aaaataaaca aataggggtt ccgcgcacat ttccccgaaa agtgccacac 6900

taaattgtaa gcgttaatat tttgttaaaa ttcgcgttaa atttttgtta aatcagctca 6960

ttttttaacc aataggccga aatcggcaaa atcccttata aatcaaaaga 7010

<210> 17
<211> 564
<212> DNA
<213> Gaussia princeps

<400> 17
atgggcgtga aggtgctgtt cgccctgatc tgcatcgccg tcgccgaggc gagatctaag 60

ccgacggaga acaacgagga cttcaacatt gtcgccgtgg cgtccaactt tgccaccacc 120

gatctggacg ccgaccgcgg caagctgccg ggcaagaagc tgcccctgga ggtcctcaag 180

gaaatggagg cgaacgcccg gaaggccggc tgcacccgcg gctgcctgat ctgcctgtcc 240

cacatcaagt gcacgccgaa gatgaagaag ttcattcccg gccggtgcca cacctacgag 300

ggcgacaagg agtccgcgca gggcggcatt ggcgaggcga tcgtggacat ccccgagatt 360

ccgggcttca aggacctgga gccgatggag cagttcatcg cccaggtgga cctgtgcgtg 420

gactgcacga ccggctgtct gaagggcctg gccaatgtgc agtgttccga cctgctcaag 480

aagtggctgc cccagcgctg tgccaccttc gccagcaaga tccagggcca ggtggacaag 540

attaagggcg ctggcggcga tacg 564

<210> 18
<211> 1134
<212> DNA
<213> Chlamydomonas reinhardtii

<400> 18
atggcgcgta ctggcgctct actcctggtc gcgctggcgc ttgcgggctg cgcgcaggct 60

tgcatctaca agttcggcac gtcgccggac tccaaggcca ccgtttcggg tgatcactgg 120

gaccatggcc tcaacggcga gaactgggag ggcaaggacg gcgcaggcaa cgcctgggtt 180

tgcaagactg gccgcaagca gtcgcccatc aacgtgcccc agtaccaggt cctggacggg 240

aagggttcca agattgccaa cggcctgcag acccagtggt cgtaccctga cctgatgtcc 300

aacggcacct cggtccaagt catcaacaac ggccacacca tccaggtgca gtggacttac 360

aactacgccg gccatgccac catcgccatc cctgccatgc acaaccagac caaccgcatc 420

gtggacgtgc tggagatgcg ccccaacgac gccgccgacc gcgtgactgc cgtgcccacc 480

cagttccact tccactccac ctcggagcac ctgctggcgg gcaagatcta tccccttgag 540

ttgcacattg tgcaccaggt gactgagaag ctggaggcct gcaagggcgg ctgcttcagc 600

gtcaccggca tcctgttcca gctcgacaac ggccccgata acgagctgct tgagcccatc 660

tttgcgaaca tgccctcgcg cgagggcacc ttcagcaacc tgccggcggg caccaccatc 720

aagctgggtg agctgctgcc cagcgaccgc gactacgtaa cgtacgaggg cagcctcacc 780

accccgccct gcagcgaggg cctgctgtgg cacgtcatga cccagccgca gcgcatcagc 840

ttcggccagt ggaaccgcta ccgcctggct gtgggcctga aggagtgcaa ctccacggag 900

accgccgcgg acgccggcca ccaccaccac caccgccgcc tgctgcacaa ccacgcgcac 960

ctggaggagg tgcctgccgc cacctccgag cccaagcact acttccgccg cgtgatgctg 1020

gccgagtccg cgaaccccga tgcctacacc tgcaaggccg ttgcctttgg ccagaacttc 1080

cgcaaccccc agtacgccaa cggccgcacc atcaagctgg cccgctatca ctaa 1134

<210> 19
<211> 378
<212> DNA
<213> Lolium perenne

<400> 19
atggacgagc agcccaacac catcagcggc agcaacaaca ccgtgcgcag cggcagcaag 60

aacgtgctgg ccggcaacga caacaccgtg atcagcggcg acaacaacag cgtgagcggc 120

agcaacaaca ccgtggtgag cggcaacgac aacaccgtga ccggcagcaa ccacgtggtg 180

agcggcacca accacatcgt gaccgacaac aacaacaacg tgagcggcaa cgacaacaac 240

gtgagcggca gcttccacac cgtgagcggc ggccacaaca ccgtgagcgg cagcaacaac 300

accgtgagcg gcagcaacca cgtggtgagc ggcagcaaca aggtggtgac cgacgcccac 360

caccaccacc accactaa 378

<210> 20
<211> 957
<212> DNA
<213> artificial sequence

<220>
<223> sequence encoding for a fusion polypeptide

<400> 20
atggcgcgta ctggcgctct actcctggtc gcgctggcgc ttgcgggctg cgcgcaggct 60

tgcagatcta agccgacgga gaacaacgag gacttcaaca ttgtcgccgt ggcgtccaac 120

tttgccacca ccgatctgga cgccgaccgc ggcaagctgc cgggcaagaa gctgcccctg 180

gaggtcctca aggaaatgga ggcgaacgcc cggaaggccg gctgcacccg cggctgcctg 240

atctgcctgt cccacatcaa gtgcacgccg aagatgaaga agttcattcc cggccggtgc 300

cacacctacg agggcgacaa ggagtccgcg cagggcggca ttggcgaggc gatcgtggac 360

atccccgaga ttccgggctt caaggacctg gagccgatgg agcagttcat cgcccaggtg 420

gacctgtgcg tggactgcac gaccggctgt ctgaagggcc tggccaatgt gcagtgttcc 480

gacctgctca agaagtggct gccccagcgc tgtgccacct tcgccagcaa gatccagggc 540

caggtggaca agattaaggg cgctggcggc gatacggata tcgacgagca gcccaacacc 600

atcagcggca gcaacaacac cgtgcgcagc ggcagcaaga acgtgctggc cggcaacgac 660

aacaccgtga tcagcggcga caacaacagc gtgagcggca gcaacaacac cgtggtgagc 720

ggcaacgaca acaccgtgac cggcagcaac cacgtggtga gcggcaccaa ccacatcgtg 780

accgacaaca acaacaacgt gagcggcaac gacaacaacg tgagcggcag cttccacacc 840

gtgagcggcg gccacaacac cgtgagcggc agcaacaaca ccgtgagcgg cagcaaccac 900

gtggtgagcg gcagcaacaa ggtggtgacc gacgcccacc accaccacca ccactaa 957

<210> 21
<211> 4
<212> PRT
<213> artificial sequence

<220>
<223> Factor Xa cleavage site

<400> 21

Ile Glu Gly Arg
1